# EUROPEAN PATENT APPLICATION

(11) **EP 4 685 221 A1**
(43) Date of publication of application: **28.01.2026**
(21) Application number: 24190886.2
(22) Date of filing: 25.07.2024
(51) Int. Cl.: C12M 1/00

(54) **USES OF ILLUMINATION ASSEMBLIES IN A PHOTOBIOREACTOR**

(71) Applicant: Gehring, Timo, 66424 Homburg (DE); Maurer, Patrick, 66540 Neunkirchen (DE)
(72) Inventor: Gehring, Timo, 66424 Homburg (DE); Maurer, Patrick, 66540 Neunkirchen (DE)
(74) Representative: Stellbrink & Partner Patentanwälte mbB

(57) **Abstract**

The present invention relates to the use of an assembly in a photobioreactor. The assembly is adapted for illuminating an interior of a container. The assembly comprises a plurality of illumination elements, wherein each illumination element comprises a light source, a power supply element, and a connector assembly for connecting the power supply element to the plurality of illumination elements. The present invention also relates to a use of a system in a photobioreactor, wherein the system is adapted for illuminating an interior of a container, wherein the system comprises a plurality of the assemblies, and to a method for illuminating an interior of a container comprising providing the system.

## Description

The present invention relates generally to the field of biological cultivation. More particularly, it relates to assemblies for facilitating biological growth, and to systems and methods for using the assemblies in a container.

The cultivation of algae may enable sustainable production of biomass and valuable materials that were previously obtained from fossil sources. Currently, 10% of the world's oil production is used for the production of plastics and as raw material for the chemical industry. Algae can make a significant contribution to establishing a carbon-based circular economy for humanity that may be, at least significantly, independent of crude oil if they can be cultivated on a large industrial scale all year round. In particular, cultivation independent of location with high yield and with green energy may be of advantage. Overexploitation of nature (e.g., algae harvesting in the sea) may also not be a sustainable option for the preservation of diversified ecosystems.

Embodiments of the present invention relate to a new type of photobioreactor for the cultivation of algae with internal illumination. The present technology may have particular advantages compared to currently available design variants of photobioreactors and may be easily scalable. As described herein, embodiments of the present technology may involve realization of internal lighting in a vessel, container, tank, or reactor for algae cultivation that may make it possible to easily transform vessels, tanks, and containers that may have been previously used for other purposes into photobioreactors. Thus, embodiments of the present technology can be particularly advantageous for algae cultivation.

Typical photobioreactors may be divided into the following categories:
1) Outdoor photobioreactors that operate with sunlight. These photobioreactors may exist as outdoor systems with the sun as the main light source, possibly with additional artificial lighting. These outdoor photobioreactors may have the disadvantage that their operating time may be limited to the duration of sunshine. In particular, they may not allow round-the-clock production/cultivation of algae. In other words, such photobioreactor systems for algae cultivation are dependent on the sun. When illuminated by the sun, the maximum production time may, thus, be limited to the duration of sunshine and may not be fully continuous all year round (8760 hours per year).
2) Indoor photobioreactors that can be illuminated from outside or inside with artificial lighting. Indoor photobioreactors may work without sunlight and only with artificial lighting, thus enabling fully continuous production 24 hours a day, 365 days a year. Thus, large-scale closed systems with internal lighting for algae cultivation may be realized with internal or external lighting.

Indoor photobioreactors with external lighting may comprise, for example, tubular reactors, pool reactors, plate reactors, and zig-zag reactors, where the lighting is installed externally into the reactor. In artificially illuminated photobioreactors with external lighting, the layer thickness of the systems, may however, be limited to 5-15 cm, making the scaling of such systems to large volumes technically complex, expensive, and inflexible.

The penetration depth of the light, generally, depends on the growth of the algae. At first, when the volume of algae in the system is low, there may be an excess of light. Once the algae culture has grown strongly, there may be too little light for continuous algae growth. In densely grown algae cultures, the culture solution may become optically dense after 5 - 15 cm, i.e. no more light may arrive after this distance.

Therefore, indoor photobioreactors may be significantly limited in size and design variety of the large-scale systems, especially with regard to the simplest possible scale-up. For example, with external lighting, the systems cannot be thick (in a direction perpendicular to the light source), as the penetration depth of light in a dense algae culture may typically be 5 - 15 cm. The scaling and cultivation of large volumes may, thus, be technically complex and/or require a lot of space.

Photobioreactors such as tubular reactors may also have other disadvantages, e.g., gas exchange due to gradient formation in the long tubes (O₂, CO₂).

Indoor photobioreactors with internal lighting that operate with permanently installed tubes (horizontal or vertical) immersed into a reaction medium of a reactor may typically contain built-in tubes that contain lighting elements. These designs may also not be easily scalable, as a large number of illumination tubes may be required with increasing container size and these must withstand the static and dynamic forces of large container dimensions or volumes. Further, the cleaning of photobioreactors with built-in parts, in particular internal fixtures such as permanently installed internal lighting units, may be complex and technically demanding.

Overall, known photobioreactor types may be technically complex and the maximum reactor size may be limited, making the production of algae complicated, in particular at large scale. A particular disadvantage of known photobioreactors using permanently installed lighting units may be the lack of scalability, especially for large cultivation volumes such as exceeding 10, 100, or 1000 m³, as a large number of lighting tubes may be required as the container size increases and these may have to withstand the static and dynamic forces of large container dimensions or volumes.

Glemser et. al., Application of light-emitting diodes (LEDs) in cultivation of phototrophic microalgae: current state and perspectives, Appl. Microbiology and Biotechnology, Vol. 100, pages 1077 - 1088 (2016), provides an overview on the application of LEDs in microalgal cultivation processes. The authors particularly focus on the use of narrow-wavelength LEDs to address fundamental as well as applied aspects of light color on algae biomass and value-added compound formation. The application of internal and external illumination systems is further reviewed together with trends in the industrial use of LED systems to intensify algae process efficiency.

Heining et. al., Internal illumination of photobioreactors via wireless light emitters: a proof of concept, Journal of Applied Phycology, Vol. 27, pages 59 - 66 (2015), discloses an illumination system based on free, suspend-able, light emitters, which are powered wirelessly by near-field resonant inductive coupling and, therefore, are able to illuminate a photobioreactor more homogeneously than external illumination systems. The authors conclude that the internal illumination via wireless light emitters results in a more uniform light distribution and a higher average light intensity in the reactor.

Heining et. al., Photobioreactors with internal illumination - A survey and comparison, Biotechnology Journal (2015), reviews different internally illuminated photobioreactors and compares them based on the ratio of illuminated surface-to-culture volume and the occupied volume by internal light-emitting elements.

US2014113362A1 discloses a closed algae cultivation system that has an algae supplier, a first conduit, at least two containers, and at least two light light-emitting devices. The algae supplier is coupled to the at least two containers via the first conduit. Each container includes at least one sidewall, a bottom and an upper lid, which together define an accommodating volume for accommodating an algal stock culture from the algae supplier. Each container is made of at least one opaque material and includes a first opening disposed on one of the at least one sidewall, and a second opening disposed at the bottom. The at least two light-emitting devices are respectively coupled to the upper lid of each container and extend therefrom along the longitudinal direction to the container such that at least a portion of the light light-emitting devices is submerged in the liquid algal stock culture.

EP2719753B1 discloses a system for illuminating the interior of a photobioreactor by means of small spheres (<= 1 cm) that may be charged from the outside by an electromagnetic field. However, in this system, the illumination may not be realized continuously, but only by light flashes, since the energy supply for electric fields cannot be realized continuously, but may only be discontinuous based on a frequency of the external electric field. Further, the external electromagnetic field may have a negative effect on algae growth. Moreover, an electromagnetic field may not be applied in a metallic stainless-steel container or on an industrial scale. Additionally, any metallic or electrical installations such as sensors and/or safety components may not be installed inside the spheres or in the reaction chamber due to interference from the external electromagnetic field. Further, due to the exponentially decreasing field strength away from the source of the electromagnetic field, spheres closer to the source may receive significantly more energy/electric field than spheres farther away. The system as disclosed in EP2719753B1 may, therefore, be difficult to scale to large containers and to metallic containers.

The present invention seeks to overcome or at least alleviate the shortcomings and disadvantages of the prior art. It is an object of the present technology to allow the realization of a photobioreactor with internal illumination. More particularly, it is an object of the present invention to allow the lighting unit to be physically separated from the container. The physical separation of the lighting unit may make it technically easier and simpler to scale up algae cultivation. In addition, empty containers may easily be transformed into photobioreactors.

According to a first aspect, the present invention relates to an assembly for illuminating an interior of a container, wherein the assembly comprises a plurality of illumination elements, wherein each illumination element comprises a light source, the assembly further comprising a power supply element, and a connector assembly for connecting the power supply element to the plurality of illumination elements. The assembly may be used, in particular, to convert a container containing a reaction medium to a photobioreactor. The assembly may be used to provide internal lighting in the photobioreactor but may not be fixed to the container as in the case of known photobioreactors with internal lighting as described above. Thus, benefits arising from separating the lighting unit from the container, as described above, may be realized.

The connector assembly may comprise a plurality of connector members; each connector member may comprise a connector cable and a plug unit, the plug unit configured to be connected to another plug unit, wherein each connector cable may be connected to an illumination element or to the power supply element.

Each connector cable may be firmly connected to the respective illumination element or to the power supply element. Firm connections may be of advantage as they may allow waterproof (as the assembly is configured to be immersed in the reaction medium) connections to be established more easily.

The plurality of illumination elements may comprise at least one illumination element to which connector cables of each of two connector members are firmly connected. In other words, at least one illumination element may be connected to two connector members. Preferably, a plurality of the plurality of illumination elements may be connected to two connector members.

The connector assembly may comprise a plurality of connector units. Each connector unit may comprise a connector cable and two connector plug units, wherein the power supply element and each of the plurality of illumination elements may comprise at least one element plug unit, wherein each element plug unit may be configured to be connected to at least one of the two connector plug units. Thus, the connector assembly may be more modular, allowing connector units to be connected to (disconnected from) an illumination element or a power supply element to add it to (remove it from) the assembly.

At least one of the plurality of illumination elements may comprise two element plug units. Preferably, a plurality, and even all, of the plurality of illumination elements may each comprise two element plug units. The two plug units may allow the illumination element to be connected to a power supply element and another illumination element or to two other illumination elements.

A first of the two element plug units may be configured to be connected to a connector plug unit of a first connector unit of the assembly, and a second of the two element plug units may be configured to be connected to a connector plug unit of a second connector unit of the assembly.

The plurality of illumination elements may comprise a distal illumination element which is connected only to one other illumination element. Alternatively, or additionally, the distal illumination element may be considered to correspond to an illumination element of the assembly at a shortest distance from a bottom of a container in which the assembly is deployed.

The plurality of illumination elements may comprise an intermediate illumination element which is connected only to two other illumination elements.

The plurality of illumination elements may comprise a proximal illumination element which is connected to the power supply element. Alternatively, or additionally, the proximal illumination element may be considered to correspond to an illumination element of the assembly at a largest distance from a bottom of a container in which the assembly is deployed.

The power supply element may be connected to only one of the plurality of illumination elements.

The connector assembly may comprise a connector cable and a plurality of connector plug units connected to the connector cable, the connector cable connected to the power supply element, wherein each of the plurality of illumination elements may comprise an element plug unit configured to be connected to a connector plug unit.

A density of the power supply element may be configured to be less than a density of any one of the plurality of illumination elements. Thus, the power supply element may remain suspended at a lower depth than any of the plurality of illumination elements when the assembly is immersed in the reaction medium. Preferably, as described further below, the density of the power supply element may be lesser than a density of the reaction medium, such that the power supply element may float at the surface of the reaction medium.

The container may comprise a reaction medium, wherein the assembly may be configured to be, at least partially, immersed in the reaction medium.

A density of the distal illumination element may be greater than a density of the reaction medium. Thus, the distal illumination element may sink into the reaction medium. Together with a density of the power supply element being lower than that of the reaction medium, this would allow the proximal and intermediate illumination elements, as described above, to sink into the reaction medium.

The power supply element may be configured to supply energy to the light source(s) of any of the plurality of illumination elements.

The connector assembly may be configured, at least in part, for energy transfer.

The connector assembly may be configured, at least in part, to conduct electricity. The connector assembly may comprise, for example, any suitable wires.

The connector assembly may be configured, at least in part, for data transfer. Data may be transferred over the same wires as electrical energy. Alternatively, or additionally, the connector assembly may comprise, for example, dedicated wires for data transfer.

A rate of data transfer through the connector assembly may be at least 1 kbps, preferably at least 100 kbps, further preferably at least 1Mbps, yet further preferably at least 100 Mbps, and even more preferably at least 1 Gbps.

The distal illumination element may be configured to be connected to a bottom of the container. Connection to the bottom of the container may be of advantage in reducing a lateral (or horizontal) movement of the assembly when deployed in the reaction medium. The connection may be accomplished by means, for example, of a hook in the bottom of the container.

The power supply element may be configured to be connected to a top or a wall of the container or a crane track or available fastening/mounting in a hall where the container may be installed. Connection to the top or wall of the container may be of advantage in reducing a lateral (or horizontal) movement of the assembly as well as in adjusting the height / depth of immersion when deployed in the reaction medium or adjusting the depth of immersion independent of a density of the assembly when fixed connections are used. The connection may be accomplished by means, for example, of a hook at the top or by using a magnet.

The assembly may be configured, at least in part, for matter transfer between any of the plurality of illumination elements and the power supply element.

The matter transfer may comprise transfer of any of a solid, a liquid, a gas, a solution, a suspension, or an emulsion. As described further below, the matter transfer may be of advantage in allowing for heat exchange.

The assembly may comprise a transfer assembly configured for the matter transfer.

The transfer assembly may comprise a tube, wherein the tube is connected between any two of the plurality of illumination elements or between one of the plurality of illumination elements and the power supply element.

The transfer assembly may comprise a plurality of tubes, each of the plurality of tubes connected between any two of the plurality of illumination elements or between one of the plurality of illumination elements and the power supply element. Preferably, the plurality of tubes may be connected between neighboring illumination elements and/or between the power supply element and the proximal illumination element as described above.

The transfer assembly may comprise a primary channel, the primary channel being connected to the power supply element.

The transfer assembly may comprise a secondary channel, the secondary channel connecting one of the plurality of illumination elements to the primary channel.

The transfer assembly may comprise a plurality of secondary channels, each of the plurality of secondary channels connecting each of a plurality of the plurality of illumination elements to the primary channel.

The transfer assembly may comprise a pump. In particular, the pump may be configured to push any of the solid, the liquid, the gas, the suspension, or the emulsion, as described above, through the tubes or the channels of the transfer assembly.

The pump may be configured to facilitate matter transfer through the tube.

The pump may be configured to facilitate matter transfer through the primary channel.

The pump may be configured to facilitate matter transfer through the secondary channel.

The distal illumination element may comprise a gas inlet device configured to provide gas to the interior of the container.

It will be understood that by the distal illumination element usually being placed in a bottom part or lower part of the container, the gas will thus be introduced to the bottom part or lower part of the container.

According to a second aspect, the present invention relates to an illumination element for illuminating an interior of a container, the illumination element comprising a light source, and wherein the illumination element is configured to be used in an assembly as described above.

The illumination element may be configured to be used in an assembly as described above, wherein the illumination element may comprise two element plug units, wherein a first of the two element plug units may be configured to be connected to a connector plug unit of a first connector unit of the assembly, and a second of the two element plug units may be configured to be connected to a connector plug unit of a second connector unit of the assembly.

The illumination element may be configured to be used in an assembly as described above, wherein the illumination element may comprise an element plug unit configured to be connected to a connector plug unit of the assembly.

The illumination element may be configured to be used in an assembly as described above, wherein the illumination element may be configured to be connected to a connector cable of a connector member of the assembly.

The illumination element may be configured to be used in an assembly as described above, wherein the illumination element may be configured to be firmly connected to a connector cable of a connector member of the assembly.

The illumination element may be configured to be used in an assembly as described above, wherein the illumination element may be configured to be connected to connector cables of each of two connector members of the assembly.

The illumination element may be configured to be firmly connected to the connector cables.

The illumination element may comprise a plurality of light sources.

A wavelength of light emitted by a first of the plurality of light sources may be different from a wavelength of light emitted by a second of the plurality of light sources. In other words, the plurality of light sources may be configured to emit light of at least two wavelengths. The ability to emit light at multiple wavelengths may be of advantage in promoting/suppressing growth of algae. For example, light at one wavelength that promotes growth may be used in an initial stage of cultivation, whereas light at another wavelength may be used to boost growth at a later stage of cultivation or to induce product formation or to suppress growth.

In particular, the different wavelengths may allow production of a value-added product with algae in a two-stage process. For example, first, algae may be allowed to grow as fast as possible to generate high amounts of biomass. Then cultivation conditions may be changed to force the algae to generate as much of the value-added product inside as possible. The algae may then be harvested or their cultivation stopped to extract the value-added product.

A wavelength of light emitted by any one of the plurality of light sources may, at least significantly, be identical to a wavelength of light emitted by any other of the plurality of light sources.

The illumination element, particularly the light source thereof, may be configured to receive energy from a power supply element of the assembly.

The energy may be received by means of a wired connection, the wired connection being provided by a connector assembly of the assembly.

The illumination element may comprise a housing.

The housing may comprise any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material. The housing may comprise material that may be rigid or flexible (inflatable/expandable/stretchable). In particular, the housing may comprise a material that may be, at least partially, inert and/or impermeable to a reaction medium of the container.

The housing may comprise a plurality of housing sections. A plurality of housing sections may be of advantage in allowing access to internal components of the illumination element for maintenance, etc. Two housing sections may allow for a simpler construction as only one joint between the two housing sections may be used to satisfy waterproofing and/or pressure constraints that may arise from immersion of the illumination element in a reaction medium of the container.

The plurality of housing sections may be configured to be releasably attached to each other.

The plurality of housing sections may be configured to be attached to each other by means of a thread connection.

The plurality of housing sections may be configured to be attached to each other by means of a bayonet connection.

For example, the plurality of housing sections may comprise two housing sections. Alternatively, the plurality of housing sections may comprise three housing sections.

The housing may comprise, at least significantly, any of a spherical, an oval, a cylindrical, an angular, a polygonal, or a platonic solid shape.

The housing may comprise two housing sections, and the housing may comprise, at least significantly, a spherical shape. Each of the two housing sections may comprise, at least significantly, a hemispherical shape.

Alternatively, as described above, the housing may comprise three housing sections comprising, for example, a substantially cylindrical section, and two substantially hemispherical sections, wherein a diameter of each of the two substantially hemispherical sections may be, at least significantly, identical to a diameter of the substantially cylindrical section. The two substantially hemispherical sections may be configured to be attached to two ends of the substantially cylindrical section so as to abut it in a direction substantially parallel to a length of the cylinder. The substantially hemispherical sections may be configured for connection to the connector assembly, whereas other components of the illumination element as described herein may be comprised within the substantially cylindrical section.

The housing may, at least partially, be transparent to the light emitted by the light source.

The container may comprise a reaction medium, wherein the illumination element may be configured to be, at least partially, submerged in the reaction medium.

The housing may, at least partially, be impermeable to the reaction medium.

The light source may be embedded in the housing.

The light source may be arranged within the housing.

The light source may be arranged on an exterior of the housing. For example, the light source may comprise a bead of LEDs that may be wrapped around the housing.

The container may comprise a reaction medium, wherein the light source arranged on the exterior of the housing may, at least partially, be inert and/or impenetrable to the reaction medium. In particular, the light source may be impenetrable to the reaction medium under pressure expected at depths at which the illumination element may be deployed. For example, a maximum expected pressure may be considered to determine suitability of the light source.

The illumination element may comprise a data processing unit.

The illumination element may comprise a control unit, wherein the control unit may be configured to control the light source.

Controlling the light source may be understood to comprise controlling a state of the light source, i.e., switching the light source on or off. Controlling may also be understood to comprise controlling a wavelength and/or an intensity and/or a flashing frequency of the light source.

The control unit may be configured to control each of the plurality of light sources.

The control unit may be configured to receive data from the data processing unit.

The control unit may be configured to control the light source based, at least in part, on the data received from the data processing unit.

The assembly may comprise an assembly as described above and the illumination element may comprise a communication unit configured to send data to and/or receive data by means of the connector assembly of the assembly.

The communication unit may be configured to receive data from the data processing unit.

The communication unit may be configured to send data to the data processing unit.

The illumination element may comprise a sensor.

The sensor may be configured to send data to the data processing unit.

The sensor may comprise a position sensor. The position sensor may be configured to determine a position of the illumination element relative to the container and/or relative to another illumination element. Further, the position sensor may be configured to determine an absolute position of the illumination element. For example, the position sensor may comprise any of a GPS sensor, a sensor based on acoustic radiation (such as a sonar) and/or on electromagnetic radiation, with an appropriate wavelength (such as a wavelength range with high permeability in aqueous media, such as visible light (400-800 nm), near infrared range (800 - 2500 nm) or radio waves (> 1mm)) to allow for transmission through a reaction medium of the container.

The sensor may comprise a temperature sensor. The temperature sensor may be configured to measure a temperature within the illumination element. The temperature sensor may be configured to measure a temperature of an environment of the illumination element. In particular, for example, two temperature sensors may be installed - one for measuring a temperature inside the illumination element and one for measuring a temperature outside. The temperature sensors may be of particular advantage in regulating a temperature of the illumination element, for example. As described later, the illumination element may also comprise a heat exchanger that may be configured to regulate a temperature inside the illumination element. The temperature sensor(s) may then be used to provide data based, at least in part, on which, the heat exchanger may regulate the temperature.

The sensor may comprise a pH sensor.

The sensor may comprise a pressure sensor.

The sensor may comprise a COz concentration sensor.

The sensor may comprise a brightness sensor. The brightness sensor may be of particular advantage in monitoring a growth of algae in an environment of the illuminated element. For example, dense growth of algae in the environment may lead to a lower sensed brightness. The illumination element, particularly the data processing unit thereof, may be configured, for example, to switch a wavelength of light emitted by the light source to suppress growth if the sensed brightness falls below a defined threshold.

The sensor may comprise at least one of a(n): O₂ concentration sensor, viscosity sensor, turbidity sensor, density sensor, conductivity sensor, impedance sensor, optical density sensor, photosynthetically active radiation (PAR) sensor, camera, microscope, IR/Raman-spectroscopy-sensor, cell potential sensor, potentiostat sensor, cell cytometry sensor, specific ion-sensor (e. g., Na⁺, Cl⁻), specific nutrient concentrations sensor, photometer sensor, proximity sensor (to detect a wall of the container), and proximity sensor / distance sensor between two illumination elements. A proximity sensor may comprise, for example, a time-of-flight sensor.

The data processing unit may comprise a sensor threshold associated with the sensor.

The data processing unit may be configured, in response to the data received from the sensor crossing the sensor threshold, to send to the communication unit data related, at least in part, thereto. The data processing unit may be further configured to perform a defined action in response to the data crossing the sensor threshold. For example, as described above, the data processing unit may switch the wavelength of light emitted by the light source in response to the brightness or cell density falling below a threshold.

The sensor may be configured to detect a signal from an environment of the illumination element, wherein the housing may be, at least partially, transparent to the signal. The signal may, in particular, comprise a radiative signal, i.e., a signal that is transmitted/received via radiation, such as electromagnetic radiation.

The container may comprise a reaction medium, and the illumination element may be configured to introduce an additive into the reaction medium. For example, the illumination element may comprise an additive dispensing unit. The additive dispensing unit may comprise, for example, a reservoir and a pump coupled to the reservoir.

The additive may comprise at least one of a gas, a nutrient, or a chemical.

The additive may comprise any of CO₂, O₃, and H₂O₂. Ozone (O₃) and hydrogen peroxide (H₂O₂) may be particularly advantageous for sanitization or oxidative stress induction purposes.

The illumination element may comprise a sampling unit configured to facilitate sample analysis. Analysis of samples may be of advantage in monitoring a progress of algae growth in the container.

The container may comprise a reaction medium, and the illumination element, particularly the sampling unit thereof, may be configured to draw and store a sample from the reaction medium. In particular, the illumination element, particularly the sampling unit thereof, may be configured to draw and store at least one or a plurality of samples from the reaction medium.

The illumination element, particularly the sampling unit thereof, may be configured to release a stored sample.

A wavelength of at least one of the plurality of light sources may be in the range defined by 150 and 1000 nm, preferably by 400 and 800 nm.

A wavelength of at least one of the plurality of light sources may be in the range defined by 150 and 450 nm, preferably by 180 and 400 nm, further preferably by 200 and 380 nm.

Alternatively, or additionally, a wavelength of at least one of the plurality of light sources may be in the range defined by 350 and 900 nm, preferably by 370 and 850 nm, further preferably by 380 and 800 nm.

Alternatively, or additionally, a wavelength of at least one of the plurality of light sources may be in the range defined by 700 and 1200 nm, preferably by 750 and 1100 nm, further preferably by 800 and 1000 nm.

Different wavelengths may provide different advantages for algae cultivation. For example, wavelengths in the range 380-800 nm may correspond to typical photosynthetically active radiation, those in the range 200-380 nm (UV light) may be of advantage for light stress induction or sanitization, whereas those in the range 800-1000 nm (Infrared light) may be of advantage for growth boosting or for stress.

In other words, different wavelengths may have different purposes, such as normal growth, switching on/off genes, inducing reactions, or simulating stress environment.

The light source may be configured to emit light with a light intensity generated on the surface of the illumination element in the range defined by 1 and 5,000 µmol/m²/s, preferably by 2 and 4,000 µmol/m²/s, further preferably by 5 and 3,000 µmol/m²/s.

The light source may comprise a light emitting diode (LED).

The LED may comprise an organic LED (OLED).

The light source may comprise a quantum dot.

An efficiency of the LED may be at least 10%, preferably at least 30%, further preferably at least 50%. The efficiency may be a function, however, of the wavelength and/or the temperature. It may be understood that the efficiencies described above may be the efficiency of the LED at least for the wavelengths and the temperatures described herein, or as known to the skilled person.

The light efficacy of modern LEDs may also be denoted in units of µmol photons per Joule (µmol/J) and for LEDs suitable for the illumination element may be in the range of 2.0 - 9.0 µmol/J (the theoretical limit in case of 100% efficacy is 1.67 µmol/J for 200 nm wavelength and 8.4 µmol/J for 1000 nm wavelength).

The light source may comprise a laser such as an organic solid-state laser.

The illumination element may be configured to fluoresce.

The illumination element may comprise a fluorescent tag, the fluorescent tag comprising fluorescent paint.

The housing may comprise an external surface, the external surface in contact with a reaction medium of the container, and an internal surface opposite the external surface.

The fluorescent tag may be attached to the external surface.

The control unit may be configured to switch an illumination state of the light source at a flashing frequency.

The flashing frequency may be in the range defined by 1 and 200 Hz, preferably by 1 and 150 Hz, further preferably by 1 and 100 Hz.

The illumination element may comprise an identification tag. The identification tag may, in particular, comprise any of an RFID, an NFC, or any other suitable tag. The identification tag may be of advantage in automatic identification of the illumination element via radiowave transmission, for example. For example, the identification tag may comprise an RFID tag configured to store a unique identifier.

The identification tag may comprise an active identification tag. Preferably, however, the identification tag may comprise a passive identification tag. An active identification tag may be understood to comprise a tag that may emit a signal of its own accord such as an active RFID tag. A passive identification tag may, on the other hand, only emit a signal in response to receiving a stimulating signal such as a passive RFID tag. An advantage of using a passive identification tag may be reduction in consumption of energy by the illumination element.

The illumination element may comprise a heat exchanger.

The illumination element may comprise a passive heat exchanger.

The illumination element may comprise an active heat exchanger. The active heat exchanger may, for example, comprise a pump, a circuit with a circulating (or working) fluid, and two or more heat exchanging surfaces. At least one of the two or more heat exchanging surfaces may, for example, be configured to exchange heat with a heat source, while at least one other of the two or more heat exchanging surfaces may be configured to exchange heat with a heat sink. Thus, heat may be transferred between the heat source and the heat sink. The heat source may comprise, for example, the illumination element, or at least a part thereof.

The heat exchanger may be configured to regulate a temperature of the illumination element. Regulating the temperature of the illumination element may be of particular advantage as a variation in temperature may affect the growth of algae in the reaction medium.

Heat exchange may also be accomplished by means of the transfer assembly as described further below.

The control unit may be further configured to control the heat exchanger.

The temperature sensor may be configured to sense the temperature in an interior of the illumination element, and wherein the heat exchanger may be configured to regulate the temperature based, at least in part, on the temperature sensed by the temperature sensor.

The illumination element may be configured to be connected to a spacer element.

The illumination element may be configured to be used in an assembly as described above, wherein the illumination element may be configured to be connected to the tube of the transfer assembly.

The illumination element may be configured to be used in an assembly as described above, wherein the illumination element may be configured to be connected to the secondary channel of the transfer assembly.

Each of the plurality of illumination elements in the assembly as described above may comprise an illumination element as described above.

According to a third aspect, the present invention relates to an illumination apparatus comprising an illumination element, the illumination element comprising a light source, and at least one connector member connected to the illumination element, wherein the at least one connector member comprises a connector cable and a plug unit, the plug unit configured to be connected to another plug unit.

The illumination apparatus may comprise two connector members connected to the illumination element, wherein each of the two connector members may comprise a connector cable and a plug unit, the plug unit configured to be connected to another plug unit.

The connector cable may comprise any of a hose, a tube, a fabric tube, or a rubber cable.

The connector cable may comprise any of a metal, a polymer, a plastic, an elastomer, a composite material, a coating, a seal, or a gasket.

The plug unit may comprise any of a metal, a polymer, a plastic, an elastomer, a composite material, a coating, a seal, a gasket, a magnetic connection, or a bayonet lock.

According to a fourth aspect, the present invention relates to a power supply element, wherein the power supply element may be configured to be used in an assembly according to any of the preceding assembly embodiments.

The power supply element may be configured to be used in an assembly as described above, wherein the power supply element may comprise an element plug unit, the element plug unit configured to be connected to a connector plug unit of a connector unit of the assembly.

The power supply element may be configured to be used in an assembly as described above, wherein the power supply element may be configured to be connected to a connector cable of a connector assembly of the assembly.

The power supply element may be configured to be used in an assembly as described above, wherein the power supply element may be configured to be connected to a connector cable of a connector member of the assembly.

The power supply element may be configured to be firmly connected to the connector cable.

The power supply element may be configured to draw energy from a local grid.

The power supply element may be configured to be connected to a connector element, wherein the connector element may comprise a connector element cable and a connector element plug unit.

The connector element cable may be firmly connected to the power supply element.

The connector element plug unit may be complementary to a socket unit of the local grid. Thus, the power supply element may draw energy from the local grid. The energy drawn from the local grid may be used to power the light source(s) of the any of the plurality of illumination elements in the assembly as described above. Use of energy from the local grid may allow for a simpler construction of the power supply element.

The power supply element may comprise a battery. Use of a battery may be of advantage if energy supply from the local grid is intermittent, or non-existent (for example, in remote locations, or where energy is extracted from renewable sources).

The battery may generally be understood to correspond to an energy storage component.

The battery may comprise a rechargeable battery.

The battery may comprise any of a Li-ion battery, a Li-polymer battery, a solid-state battery, a super capacitor battery, an Ni-Cd battery, an Ni-Metal Hydride battery, a redox flow battery, a sodium ion battery, and a lithium-sulphur solid-state battery.

The battery may be configured to be charged by the energy drawn from the local grid. In other words, the power supply element may comprise a battery as well as be configured to draw energy from the local grid.

The battery may comprise a replaceable battery.

The power supply element may comprise a power supply element data processing unit.

The power supply element may comprise a power supply element memory unit.

The power supply element memory unit may be configured to communicate with the power supply element data processing unit.

The power supply element may comprise a power supply element communication unit.

The power supply element communication unit may be configured to communicate with the power supply element data processing unit.

The power supply element communication unit may be configured to communicate with an external device and/or element. The external device may comprise, for example, a remote server or a remote computer. The external element may comprise, for example, one of the plurality of illumination elements of the assembly.

The power supply element may be configured to be used with an assembly as described above, wherein at least one of the plurality of illumination elements may comprise an illumination element as described above, wherein the power supply element communication unit may be configured to communicate with at least one illumination element by means of the connector assembly.

The power supply element, particularly the power supply element communication unit thereof, may be configured to receive data from the at least one illumination element.

The power supply element communication unit may be configured to send the received data, or at least a part thereof, to the external device and/or element.

The power supply element communication unit may be configured to send the data to the power supply element data processing unit.

The power supply element data processing unit may be configured to perform a defined action based, at least in part, on the data received. For example, the data received may relate, at least in part, to a temperature within the at least one illumination element. The power supply element data processing unit may be configured, in response, to activate an active heat exchanger of the at least one illumination element, or, at least to send data related thereto, to the at least one illumination element.

The power supply element data processing unit may be configured to send the data received to the power supply element memory unit. The data may be retrieved from the power supply element memory unit later for analysis, for example. Alternatively, or additionally, the power supply element data processing unit may be configured to facilitate sending of the stored data to the external device and/or element at a later instant of time. For example, the data may be sent when a connectivity to the external device and/or element is determined to be higher than a pre-defined threshold. Or, data may be sent when a size of the stored data exceeds a pre-defined threshold.

The at least one illumination element may comprise an illumination element as described above, wherein the data received may comprise data measured by the sensor, or at least data related thereto.

The power supply element may be configured to send data to the at least one illumination unit.

The power supply element may be configured to be used in an assembly as described above, wherein a density of the power supply element is less than a density of the reaction medium, or at least a part thereof.

The power supply element may comprise a housing.

The housing may comprise any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material.

The material may be rigid or flexible (inflatable/expandable/stretchable).

The housing may comprise a plurality of housing sections, such as two housing sections.

The two housing sections may be configured to be releasably attached to each other.

The two housing sections may be configured to be attached to each other by means of a thread connection.

The two housing sections may be configured to be attached to each other by means of a bayonet connection.

The housing may comprise, at least significantly, any of a spherical, an oval, a cylindrical, an angular, a platonic solid, or a polygonal shape.

The housing may comprise, at least significantly, a spherical shape, wherein each of the two housing sections may comprise, at least significantly, a hemispherical shape.

The power supply element may be configured to be used in an assembly as described above, wherein the housing may be, at least partially, impermeable and/or inert to the reaction medium.

The assembly may comprise an assembly as described above, wherein a storage capacity of the battery may be based, at least in part, on the total illuminated (illuminable) surface area of the plurality of illumination elements in the assembly.

A storage capacity of the battery may be in the range defined by 0.005 and 50 kWh per m² of illuminated surface area of the plurality of illumination elements connected to the power supply element, preferably by 0.1 and 25 kWh per m², further preferably by 1 and 10 kWh per m².

The power supply element may be configured to be connected to a spacer element.

The power supply element may be configured to be used in an assembly as described above, wherein the power supply element may be configured to be connected to the tube of the transfer assembly.

The power supply element may be configured to be used in an assembly as described above, wherein the power supply element may be configured to be connected to the primary channel of the transfer assembly.

The power supply element may comprise a power supply element as described above, wherein the connector assembly may be configured to transfer energy drawn by the power supply element from the local grid to any of the plurality of illumination elements.

The power supply element may comprise a light source.

The power supply element may comprise a plurality of light sources.

At least one of the plurality of light sources may be configured to indicate a status of the assembly.

The power supply element may be configured to be used in an assembly as described above, wherein at least one of the plurality of light sources may be configured for illumination of the reaction medium, or at least a part thereof.

For example, the power supply element may comprise two light sources. A first of the two light sources may be arranged, for example, in a volume of the power supply element that floats above a surface of the reaction medium, as described above, and may be of advantage in indicating a status of the assembly such as active or inactive. A second of the two light sources may be arranged, for example, closer to a surface of the reaction medium than the first light source. The second light source may be of advantage in illuminating the reaction medium, or at least a part thereof, thus optimizing the growth of algae in the container.

The power supply element may comprise a heat exchanger. The heat exchanger may comprise an active and/or a passive heat exchanger similar to the active and/or passive heat exchanger comprised by the illumination element as described herein. The heat exchanger may be configured to exchange heat

According to a fifth aspect, the present invention relates to a power apparatus comprising a power supply element and at least one connector member connected to the power supply element, wherein the at least one connector member comprises a connector cable and a plug unit, the plug unit configured to be connected to another plug unit.

The power apparatus may further comprise a connector element connected to the power supply element, wherein the connector element may comprise a connector element cable and a connector element plug unit, the connector element plug unit configured to be connected to a socket unit of a local grid.

The connector cable may comprise any of a hose, a tube, a fabric tube, or a rubber cable.

The connector cable may comprise any of a metal, a polymer, a plastic, an elastomer, a composite material, a coating, a seal, or a gasket.

The plug unit may comprise any of a metal, a polymer, a plastic, an elastomer, a composite material, a coating, a seal, a gasket, a magnetic connection, or a bayonet lock.

The connector element cable may comprise any of a hose, a tube, a fabric tube, or a rubber cable.

The connector element cable may comprise any of a metal, a polymer, a plastic, an elastomer, a composite material, a coating, a seal, or a gasket.

The connector element plug unit may comprise any of a metal, a polymer, a plastic, an elastomer, a composite material, a coating, a seal, a gasket, a magnetic connection, or a bayonet lock.

The power supply element may comprise a power supply element as described above, wherein the connector assembly may be configured to transfer energy stored in the battery to any of the plurality of illumination elements.

The control unit of the at least one illumination element to which the power supply element as described above may send data may be configured to control the light source based, at least in part, on the data received from the power supply element.

The power supply element comprised in the assembly as described above may comprise a power supply element as described above.

A density of assembly may be less than a density of the reaction medium.

A ratio of the density of the assembly to a density of the reaction medium may be based, at least in part, on a ratio of a volume of the assembly completely immersed in the reaction medium to a total volume of the assembly.

According to a sixth aspect, the present invention relates to a system for illuminating an interior of a container, wherein the system comprises a plurality of assemblies, and wherein each of the plurality of assemblies comprises an assembly as described above.

The system may comprise a spacer assembly configured to allow an arrangement of the plurality of assemblies in a volume of the container.

The spacer assembly, or at least a part thereof, may be configured to maintain a non-zero distance between any two of the plurality of assemblies.

The spacer assembly may comprise at least one or a plurality of spacer elements, wherein each spacer element is connected to an illumination element or to a power supply element of an assembly of the plurality of assemblies.

At least one of the at least one or plurality of spacer elements may be connected to two illumination elements, wherein a first of the two illumination elements is comprised in a first of the plurality of assemblies, and a second of the two illumination elements is comprised in a second of the plurality of assemblies.

At least one of the at least one or plurality of spacer elements may be connected to two power supply elements, wherein a first of the two power supply elements is comprised in a first of the plurality of assemblies, and a second of the two power supply elements is comprised in a second of the plurality of assemblies.

At least one of the at least one or plurality of spacer elements may comprise any of a rope, a line spacer, a chain, a rigid rod, a rigid tube, a metal rod, a plastic rod, a flexible hose, buoyant foam, a ring, or a sphere, such as a permeable sphere.

Each spacer element may be firmly connected to the respective illumination element or to the power supply element.

Each spacer element may be releasably connected to the respective illumination element or to the power supply element.

Each of the plurality of illumination elements in each of the plurality of assemblies may comprise an illumination element as described above, wherein the housing may comprise, at least significantly, a spherical shape, and wherein a radius of the housing may be, at least significantly, identical for each of the plurality of illumination elements. In other words, the system may comprise a plurality of illumination elements across the plurality of assemblies, each of the plurality of illumination elements comprising, at least significantly a spherical housing, and a radius of the spherical housing may be, at least significantly, identical for each of the plurality of illumination elements across the plurality of assemblies.

The power supply element in each of the plurality of assemblies may comprise a power supply element as described above, wherein the housing may comprise, at least significantly, a spherical shape, and wherein a radius of the housing may be, at least significantly, identical for each power supply element. In other words, the system may comprise a plurality of power supply elements across the plurality of assemblies, each of the plurality of power supply elements comprising, at least significantly a spherical housing, and a radius of the spherical housing may be, at least significantly, identical for each of the plurality of power supply elements across the plurality of assemblies.

According to a seventh aspect, the present invention relates to a use of an assembly as described above or a system as described above in a photobioreactor.

The photobioreactor may comprise the container and the container may comprise a reaction medium.

The container may comprise a volume at least as large as 0.1 m³, preferably at least as large as 0.5 m³, further preferably at least as large as 1 m³.

The illumination element may comprise a housing made of metal.

The container may be used for algae cultivation, cultivation of phototrophic organisms, cyanobacteria, corals, underwater plants, moss, microalgae, macroalgae, artificial photosynthetic systems, or biomass production.

The algae may comprise any of Chlorella vulgaris, Spirulina platensis, Haematococcus pluvialis, Phaeodactylum tricornutum, Nostoc muscorum, Chlamydomonas reinhardtii, Dunaliella salina, Nannochloropsis, Scenedesmus, Botryococcus braunii, Porphyridium, Synechocystis, Synechococcus, and co-cultures of phototrophic and non-phototrophic organisms.

The container may comprise an agitator, such as a pump, a flow pump, or a gas circulator.

The container may comprise at least one metal appliance.

The container may comprise any of a gas inlet, a gas outlet, a sensor, or harvesting equipment.

The container may be configured for dosing and/or sampling of a reaction medium in the container.

The container may be configured to allow the distal illumination element of the assembly as described above to be connected to a bottom of the container. For example, the container may comprise a hook on the bottom to which a latch from the distal illumination element may be connected. Alternatively, the container may comprise a socket on the bottom into which a plug of the distal illumination element may be fit. Generally, it may be understood, that any suitable means for connecting the distal illumination element, and thus the assembly, to the container may be provided.

According to an eighth aspect, the present invention relates to a method for illuminating an interior of a container, wherein the method comprises providing an assembly as described above or a system as described above. In other words, the method may comprise providing one or a plurality of assemblies, each of the one or plurality of assemblies comprising an assembly as described above.

The method may comprise arranging the assembly and/or the plurality of assemblies in a volume of the container such that the assembly and/or the plurality of assemblies define a number density of illumination elements in the container.

The method may comprise arranging the assembly and/or the plurality of assemblies such that the number density of illumination elements is, at least significantly, identical to a desired number density of illumination elements in the container. In particular, the desired number density of illumination elements may be achieved not only by adding and/or removing illumination elements, as described further below, but also by adding and/or removing one or more of the at least one or plurality of assemblies.

The container may comprise a reaction medium, and the desired number density may be based, at least in part, on a total illuminated surface area per unit volume of the reaction medium.

The total illuminated surface area may be in the range defined by 1 and 30 m², preferably by 1 and 25 m², further preferably by 1 and 20 m², for 1000 L volume of the reaction medium.

The method may comprise changing the number density of illumination elements in the container.

Changing the number density of illumination elements may comprise changing the number of illumination elements in at least one assembly.

Changing the number density of illumination elements may comprise changing the number of illumination elements in a plurality of assemblies.

Changing the number of illumination elements in a defined assembly of the at least one assembly or of the plurality of assemblies may comprise adding an illumination element to or removing an illumination element from the defined assembly.

The defined assembly may comprise an assembly as described above, wherein adding an illumination element to (removing an illumination from) the defined assembly may comprise connecting (disconnecting) a plug unit of a connector member, the connector cable of which is connected to the illumination element, to (from) the plug unit of another connector member, the connector cable of which is connected to another illumination element or to the power supply element.

The defined assembly may comprise an assembly as described above, wherein adding an illumination element to (removing an illumination from) the defined assembly may comprise connecting (disconnecting) a plug unit of each connector member, the connector cable of which is connected to the illumination element, to (from) the plug unit of another connector member, the connector cable of which is connected to another illumination element or to the power supply element.

The defined assembly may comprise an assembly as described above, wherein adding an illumination element to (removing an illumination from) the defined assembly may comprise connecting (disconnecting) an element plug unit of the illumination element to (from) a connector plug unit of a connector unit.

The defined assembly may comprise an assembly as described above, wherein adding an illumination element to (removing an illumination from) the defined assembly may comprise connecting (disconnecting) each of the two element plug units of the illumination element to (from) a connector plug unit of a connector unit.

The method may comprise removing an illumination element from the defined assembly, and wherein a result of removing the illumination element is disconnection, from the assembly, of at least one illumination element.

An illumination element may be understood to be disconnected from the assembly if energy cannot be transferred from the power supply element to the illumination element.

The method may comprise connecting the disconnected illumination element to the assembly.

In particular, when an intermediate illumination element, as described above, is removed from the defined assembly, a part of the defined assembly (comprising the illumination elements between the removed illumination element and the distal illumination element and the distal illumination element) may get disconnected from the defined assembly. The removal of the illumination element may be followed, then, by reconnecting the disconnected part of the defined assembly to the defined assembly, for example, by reconnecting the illumination element of the disconnected part that was previously connected to the removed illumination element to an illumination element of the defined assembly.

The defined assembly may comprise an assembly as described above, wherein adding an illumination element to (removing an illumination from) the defined assembly may comprise connecting (disconnecting) an element plug unit of the illumination element to (from) a connector plug unit.

The method may comprise changing the number density based, at least in part, on a total illuminated surface area per unit volume of the reaction medium.

The method may comprise changing the total illuminated surface area per unit volume of the reaction medium by a factor less than 30, preferably less than 25, further preferably less than 20.

The method may comprise maintaining the number density constant for at least 10 minutes, preferably at least 30 minutes, further preferably at least 1 hour and then changing the number density.

The container may be used for algae cultivation, wherein the method may comprise changing the number density based, at least in part, on a level of growth of the algae or formation of a product in the container.

The container may be used for algae cultivation, wherein the method may comprise changing the number density based, at least in part, on a type of the algae.

The assembly may comprise a power supply element as described above, wherein the method may comprise detecting a low, or no, remaining battery capacity of the battery.

The method may comprise, in response to the detection, replacing the power supply element with another power supply element comprising a power supply element according to any of the preceding power supply element embodiments.

The method may comprise charging the battery.

The method may comprise replacing the battery of the power supply element.

The method may comprise charging the replaced battery.

The container may comprise a reaction medium, wherein the method may comprise immersing, at least partially, the assembly in the reaction medium.

The assembly may comprise a plurality of illumination elements, and the method may comprise cleaning at least one of the plurality of illumination elements.

Cleaning the at least one illumination element may comprise retrieving, from the reaction medium, the assembly.

Cleaning the at least illumination element may comprise removing, from the assembly, the at least one illumination element, and retrieving the at least one illumination element.

The assembly may comprise a plurality of illumination elements, and the method may comprise wrapping each of the plurality of illumination elements in a protective film before immersing in the reaction medium.

Cleaning at least one of the plurality of illumination elements may comprise changing the protective film wrapped around the at least one illumination element.

The protective film may be, at least partially, inert and/or impermeable to the reaction medium.

The assembly may comprise an illumination element as described above, and the method may comprise determining if the sampling unit comprises a sample.

The method may comprise, in response to the determination that the sampling unit comprises a sample, retrieving the sample from the illumination element.

Changing the number of illumination elements may comprise adding an illumination element to the defined assembly, wherein the defined assembly may comprise a power supply element as described above, wherein the method may further comprise determining if a storage capacity of the battery is sufficient.

Sufficiency of the battery capacity may be determined based, at least in part, for example, on the total illuminated surface area after the addition of the illumination element.

The power supply element may comprise a power supply element as described above, wherein the method may comprise, in response to determining the storage capacity of the battery to be insufficient, replacing the battery with another battery of higher storage capacity.

The method may comprise providing a system as described above, wherein the method may further comprise realizing, using the spacer assembly, a defined arrangement of the plurality of assemblies in a volume of the container.

The method may comprise providing a system as described above, wherein the method may comprise realizing, at least significantly, a close-packing of the plurality of illumination and power supply elements.

The illumination element may be configured to be used in the use as described above and/or the method as described above.

The power supply element may be configured to be used in the use as described above and/or the method as described above.

The present invention is also described by the following numbered embodiments.

Below, assembly embodiments will be discussed. These are abbreviated by the letter "A" followed by a number. Whenever reference is herein made to the assembly embodiments, the following embodiments are meant.

A1. An assembly for illuminating an interior of a container, wherein the assembly comprises
a plurality of illumination elements (10), wherein each illumination element (10) comprises a light source,
a power supply element (20), and
a connector assembly (30) for connecting the power supply element (20) to the plurality of illumination elements (10).

A2. The assembly according to the preceding embodiment,
wherein the connector assembly (30) comprises a plurality of connector members (32), wherein each connector member (32) comprises a connector cable (322) and a plug unit (324), the plug unit (324) configured to be connected to another plug unit, wherein each connector cable (322) is connected to an illumination element (10) or to the power supply element (20).

A3. The assembly according to the preceding embodiment, wherein each connector cable (322) is firmly connected to the respective illumination element (10) or to the power supply element (20).

A4. The assembly according to any of the 2 preceding embodiments, wherein the plurality of illumination elements comprises at least one illumination element to which connector cables of each of two connector members are connected.

A5. The assembly according to embodiment A1,
wherein the connector assembly comprises a plurality of connector units (34), wherein each connector unit (34) comprises a connector cable and two connector plug units,
wherein the power supply element (20) and each of the plurality of illumination elements (10) comprises at least one element plug unit, wherein each element plug unit is configured to be connected to at least one of the two connector plug units.

A6. The assembly according to the preceding embodiment, wherein at least one of the plurality of illumination elements comprises two element plug units.

A7. The assembly according to the preceding embodiment, wherein a first of the two element plug units is configured to be connected to a connector plug unit of a first connector unit of the assembly, and a second of the two element plug units is configured to be connected to a connector plug unit of a second connector unit of the assembly.

A8. The assembly according to any of the preceding assembly embodiments, wherein the plurality of illumination elements comprises a distal illumination element which is connected only to one other illumination element.

A9. The assembly according to any of the preceding assembly embodiments and with the features of any of embodiments A4, and A6, wherein the plurality of illumination elements comprises an intermediate illumination element which is connected only to two other illumination elements.

A10. The assembly according to any of the preceding assembly embodiments, wherein the plurality of illumination elements comprises a proximal illumination element which is connected to the power supply element.

A11. The assembly according to any of the preceding assembly embodiments, wherein the power supply element is connected to only one of the plurality of illumination elements.

A12. The assembly according to embodiment A1,
wherein the connector assembly (30) comprises a connector cable (36) and a plurality of connector plug units (38) connected to the connector cable (36), wherein the connector cable is connected to the power supply element (20),
wherein each of the plurality of illumination elements (10) comprises an element plug unit configured to be connected to a connector plug unit (38).

A13. The assembly according to any of the preceding assembly embodiments, wherein a density of the power supply element is configured to be less than a density of any one of the plurality of illumination elements.

A14. The assembly according to any of the preceding assembly embodiments, wherein the container comprises a reaction medium, wherein the assembly is configured to be, at least partially, immersed in the reaction medium.

A15. The assembly according to the preceding embodiment and with the features of embodiment A8, wherein a density of the distal illumination element is greater than a density of the reaction medium.

A16. The assembly according to any of the preceding assembly embodiments, wherein the power supply element is configured to supply energy to the light source(s) of any of the plurality of illumination elements.

A17. The assembly according to any of the preceding assembly embodiments, wherein the connector assembly is configured, at least in part, for energy transfer.

A18. The assembly according to the preceding embodiment, wherein the connector assembly is configured, at least in part, to conduct electricity.

A19. The assembly according to any of the preceding assembly embodiments, wherein the connector assembly is configured, at least in part, for data transfer.

A20. The assembly according to the preceding embodiment, wherein a rate of data transfer through the connector assembly is at least 1 kbps, preferably at least 100 kbps, further preferably at least 1Mbps, yet further preferably at least 100 Mbps, and even more preferably at least 1 Gbps.

A21. The assembly according to any of the preceding assembly embodiments and with the features of embodiment A8, wherein the distal illumination element is configured to be connected to a bottom of the container.

A22. The assembly according to any of the preceding assembly embodiments, wherein the assembly is configured, at least in part, for matter transfer between any of the plurality of illumination elements and the power supply element.

A23. The assembly according to the preceding embodiment, wherein the matter transfer comprises transfer of any of a solid, a liquid, a gas, a solution, a suspension, or an emulsion.

A24. The assembly according to any of the 2 preceding embodiments, wherein the assembly comprises a transfer assembly configured for the matter transfer.

A25. The assembly according to the preceding embodiment, wherein the transfer assembly comprises a tube, wherein the tube is connected between any two of the plurality of illumination elements or between one of the plurality of illumination elements and the power supply element.

A26. The assembly according to the preceding embodiment, wherein the transfer assembly comprises a plurality of tubes, each of the plurality of tubes connected between any two of the plurality of illumination elements or between one of the plurality of illumination elements and the power supply element.

A27. The assembly according to embodiment A24, wherein the transfer assembly comprises a primary channel, the primary channel being connected to the power supply element.

A28. The assembly according to the preceding embodiment, wherein the transfer assembly comprises a secondary channel, the secondary channel connecting one of the plurality of illumination elements to the primary channel.

A29. The assembly according to the preceding embodiment, wherein the transfer assembly comprises a plurality of secondary channels, each of the plurality of secondary channels connecting each of a plurality of the plurality of illumination elements to the primary channel.

A30. The assembly according to any of the preceding assembly embodiments and with the features of embodiment A24, wherein the transfer assembly comprises a pump.

A31. The assembly according to the preceding embodiment and with the features of embodiment A25, wherein the pump is configured to facilitate matter transfer through the tube.

A32. The assembly according to the penultimate embodiment and with the features of embodiment A27, wherein the pump is configured to facilitate matter transfer through the primary channel.

A33. The assembly according to embodiment A30, and with the features of embodiment A28, optionally with the features of the preceding embodiment, wherein the pump is configured to facilitate matter transfer through the secondary channel.

A34. The assembly according to any of the preceding embodiments and with the features of embodiment A8, wherein the distal illumination element comprises a gas inlet device configured to provide gas to the interior of the container.

It will be understood that by the distal illumination element usually being placed in a bottom part or lower part of the container, the gas will thus be introduced to the bottom part or lower part of the container.

Below illumination element embodiments will be discussed. These are abbreviated by the letter "I" followed by a number. Whenever reference is herein made to the illumination element embodiments, the following embodiments are meant.

I1. An illumination element (10) for illuminating an interior of a container (40), wherein the illumination element comprises a light source (110), and wherein the illumination element is configured to be used in an assembly according to any of the preceding assembly embodiments.

I2. The illumination element according to embodiment I1, wherein the illumination element is configured to be used in an assembly according to embodiment A5, wherein the illumination element comprises an element plug unit configured to be connected to a connector plug unit of a connector unit of the assembly.

I3. The illumination element according to the preceding embodiment, wherein the illumination element is configured to be used in an assembly according to embodiment A6, wherein the illumination element comprises two element plug units, wherein a first of the two element plug units is configured to be connected to a connector plug unit of a first connector unit of the assembly, and a second of the two element plug units is configured to be connected to a connector plug unit of a second connector unit of the assembly.

I4. The illumination element according to embodiment I1, wherein the illumination element is configured to be used in an assembly according to embodiment A12, wherein the illumination element comprises an element plug unit configured to be connected to a connector plug unit of the assembly.

I5. The illumination element according to embodiment I1, wherein the illumination element is configured to be used in an assembly according to embodiment A2, wherein the illumination element is configured to be connected to a connector cable of a connector member of the assembly.

I6. The illumination element according to the preceding embodiment, wherein the illumination element is configured to be used in an assembly according to embodiment A3, wherein the illumination element is configured to be firmly connected to a connector cable of a connector member of the assembly.

I7. The illumination element according to any of the 2 preceding embodiments, wherein the illumination element is configured to be used in an assembly according to embodiment A4 wherein the illumination element is configured to be connected to connector cables of each of two connector members of the assembly.

I8. The illumination element according to the preceding embodiment, wherein the illumination element is configured to be firmly connected to the connector cables.

I9. The illumination element according to any of the preceding illumination element embodiments, wherein the illumination element comprises a plurality of light sources.

I10. The illumination element according to the preceding embodiment, wherein a wavelength of light emitted by a first of the plurality of light sources is different from a wavelength of light emitted by a second of the plurality of light sources.

I11. The illumination element according to the penultimate embodiment, wherein a wavelength of light emitted by any one of the plurality of light sources is, at least significantly, identical to a wavelength of light emitted by any other of the plurality of light sources.

112. The illumination element according to any of the preceding illumination element embodiments, wherein the light source is configured to receive energy from a power supply element of the assembly.

I13. The illumination element according to the preceding embodiment, wherein the energy is received by means of a wired connection, the wired connection being provided by a connector assembly of the assembly.

I14. The illumination element according to any of the preceding embodiments, wherein the illumination element comprises a housing (134).

115. The illumination element according to the preceding embodiment, wherein the housing comprises any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material.

The material may be rigid or flexible (inflatable/expandable/stretchable).

116. The illumination element according to any of the 2 preceding embodiments, wherein the housing comprises a plurality of housing sections.

I17. The illumination element according to the preceding embodiment, wherein at least two of the plurality of housing sections are configured to be releasably attached to each other.

I18. The illumination element according to the preceding embodiment, wherein at least two of the plurality of housing sections are configured to be attached to each other by means of a thread connection.

I19. The illumination element according to any of the 2 preceding embodiments, wherein at least two of the plurality of housing sections are configured to be attached to each other by means of a bayonet connection.

I20. The illumination element according to any of the preceding illumination element embodiments and with the features of embodiment I14, wherein the housing comprises, at least significantly, any of a spherical, an oval, a cylindrical, an angular, a platonic solid, or a polygonal shape.

I21. The illumination element according to the preceding embodiment and with the features of embodiment I16, wherein the housing comprises two housing sections, and wherein the housing comprises, at least significantly, a spherical shape, and wherein each of the two housing sections comprises, at least significantly, a hemispherical shape.

I22. The illumination element according to any of the preceding illumination element embodiments and with the features of embodiment I14, wherein the housing is, at least partially, transparent to the light emitted by the light source.

I23. The illumination element according to any of the preceding illumination element embodiments, wherein the container comprises a reaction medium, and wherein the illumination element is configured to be, at least partially, submerged in the reaction medium.

I24. The illumination element according to the preceding embodiment and with the features of embodiment I14, wherein the housing is, at least partially, impermeable to the reaction medium.

I25. The illumination element according to any of the preceding illumination element embodiments and with the features of embodiment I14, wherein the light source is embedded in the housing.

I26. The illumination element according to any of the preceding illumination element embodiments and with the features of embodiment I14 but without the features of the preceding embodiment, wherein the light source is arranged within the housing.

I27. The illumination element according to any of the preceding illumination element embodiments and with the features of embodiment I14 but without the features of any of the 2 preceding embodiments, wherein the light source is arranged on an exterior of the housing.

I28. The illumination element according to the preceding embodiment, wherein the container comprises a reaction medium, and wherein the light source is, at least partially, inert and/or impermeable to the reaction medium.

I29. The illumination element according to any of the preceding illumination element embodiments, wherein the illumination element comprises a data processing unit (174).

I30. The illumination element according to any of the preceding illumination element embodiments, wherein the illumination element comprises a control unit (172), wherein the control unit is configured to control the light source.

Controlling the light source may be understood to comprise controlling a state of the light source, i.e., switching the light source on or off. Controlling may also be understood to comprise controlling a wavelength and/or an intensity and/or a flashing frequency of the light source.

I31. The illumination element according to the preceding embodiment and with the features of embodiment I9, wherein the control unit is configured to control each of the plurality of light sources.

I32. The illumination element according to any of the 2 preceding embodiments and with the features of embodiment I29, wherein the control unit is configured to receive data from the data processing unit.

I33. The illumination element according to the preceding embodiment, wherein the control unit is configured to control the light source based, at least in part, on the data received from the data processing unit.

I34. The illumination element according to any of the preceding illumination element embodiments, wherein the assembly comprises an assembly according to embodiment A19, and wherein the illumination element further comprises a communication unit (176) configured to send and/or receive data by means of the connector assembly of the assembly.

I35. The illumination element according to the preceding embodiment and with the features of embodiment I29, wherein the communication unit is configured to receive data from and/or send data to the data processing unit.

I36. The illumination element according to any of the preceding illumination element embodiments, wherein the illumination element further comprises a sensor (180).

I37. The illumination element according to the preceding embodiment and with the features of embodiment I29, wherein the sensor is configured to send data to the data processing unit.

I38. The illumination element according to any of the preceding illumination element embodiments and with the features of embodiment I36, wherein the sensor comprises a position sensor.

I39. The illumination element according to any of the preceding illumination element embodiments and with the features of embodiment I36, wherein the sensor comprises a temperature sensor.

I40. The illumination element according to the preceding embodiment, wherein the temperature sensor is configured to measure a temperature within the illumination element.

I41. The illumination element according to any of the 2 preceding embodiments, wherein the temperature sensor is configured to measure a temperature of an environment of the illumination element.

I42. The illumination element according to any of the preceding illumination element embodiments and with the features of embodiment I36, wherein the sensor comprises a pH sensor.

I43. The illumination element according to any of the preceding illumination element embodiments and with the features of embodiment I36, wherein the sensor comprises a pressure sensor.

I44. The illumination element according to any of the preceding illumination element embodiments and with the features of embodiment I36, wherein the sensor comprises a COz concentration sensor.

I45. The illumination element according to any of the preceding illumination element embodiments and with the features of embodiment I36, wherein the sensor comprises a brightness sensor.

I46. The illumination element according to any of the preceding illumination element embodiments and with the features of embodiment I36, wherein the sensor comprises at least one of an O₂ concentration sensor, a viscosity sensor, a turbidity sensor, a density sensor, a conductivity sensor, an impedance sensor, an optical density sensor, a photosynthetically active radiation (PAR) sensor, a camera, a microscope, an IR/Raman-spectroscopy-sensor, a cell potential sensor, a potentiostat sensor, a cell cytometry sensor, a specific ion-sensor (e. g., Na⁺, Cl⁻), a specific nutrient concentrations sensor, a photometer sensor, a proximity sensor (to detect a wall of the container), a proximity sensor and/or a distance sensor between two illumination elements.

I47. The illumination element according to any of the preceding illumination element embodiments and with the features of embodiments I29, and I36, wherein the data processing unit comprises a sensor threshold associated with the sensor.

I48. The illumination element according to the preceding embodiment and with the features of embodiments I35, and I37, wherein the data processing unit is configured, in response to the data received from the sensor crossing the sensor threshold, to send to the communication unit data related, at least in part, thereto.

I49. The illumination element according to any of the preceding illumination embodiments and with the features of embodiments I14, and I36, wherein the sensor is configured to detect a radiative signal from an environment of the illumination element, and wherein the housing is, at least partially, transparent to the radiative signal.

I50. The illumination element according to any of the preceding illumination element embodiments, wherein the container comprises a reaction medium, and wherein the illumination element is configured to introduce an additive into the reaction medium. I51. The illumination element according to the preceding embodiment, wherein the additive comprises at least one of a gas, a nutrient, or a chemical.

I52. The illumination element according to any of the 2 preceding embodiments, wherein the additive comprises any of CO₂, O₃, and H₂O₂.

I53. The illumination element according to any of the preceding illumination element embodiments, wherein the illumination element comprises a sampling unit (148) configured to facilitate sample analysis.

I54. The illumination element according to the preceding embodiment, wherein the container comprises a reaction medium, and wherein the illumination element, particularly the sampling unit thereof, is configured to draw and store a sample from the reaction medium.

I55. The illumination element according to any of the 2 preceding embodiments, wherein the illumination element, particularly the sampling unit thereof, is configured to release a stored sample.

I56. The illumination element according to any of the preceding illumination element embodiments and with the features of any of embodiments I10, and I11, wherein a wavelength of at least one of the plurality of light sources is in the range defined by 150 and 450 nm, preferably by 180 and 400 nm, further preferably by 200 and 380 nm.

I57. The illumination element according to any of the preceding illumination element embodiments and with the features of any of embodiments I10, and I11, wherein a wavelength of at least one of the plurality of light sources is in the range defined by 350 and 900 nm, preferably by 370 and 850 nm, further preferably by 380 and 800 nm.

I58. The illumination element according to any of the preceding illumination element embodiments and with the features of any of embodiments I10, and I11, wherein a wavelength of at least one of the plurality of light sources is in the range defined by 700 and 1200 nm, preferably by 750 and 1100 nm, further preferably by 800 and 1000 nm.

I59. The illumination element according to any of the preceding illumination element embodiments, wherein the light source is configured to emit light with a light intensity generated on the surface of the illumination element in the range defined by 1 and 5000 µmol/m²/s, preferably by 2 and 4000 µmol/m²/s, further preferably by 5 and 3000 µmol/m²/s.

I60. The illumination element according to any of the preceding illumination element embodiments, wherein the light source comprises a light emitting diode (LED).

I61. The illumination element according to the preceding embodiment, wherein the LED comprises an organic LED (OLED).

I62. The illumination element according to any of the preceding illumination element embodiments, wherein the light source comprises a quantum dot.

I63. The illumination element according to any of the preceding illumination element embodiments and with the features of embodiment I60, wherein an efficiency of the LED is at least 10%, preferably at least 30%, further preferably at least 50%.

I64. The illumination element according to any of the preceding illumination embodiments, wherein the light source comprises a laser such as an organic solid-state laser.

I65. The illumination element according to any of the preceding illumination element embodiments, wherein the illumination element is configured to fluoresce.

I66. The illumination element according to the preceding embodiment, wherein the illumination element comprises a fluorescent tag (152), the fluorescent tag comprising fluorescent paint.

I67. The illumination element according to any of the preceding illumination element embodiments and with the features of embodiment I14, wherein the housing comprises an external surface, the external surface in contact with a reaction medium of the container, and an internal surface opposite the external surface.

I68. The illumination element according to the preceding embodiment and with the features of embodiment I66, wherein the fluorescent tag is attached to the external surface.

I69. The illumination element according to any of the preceding illumination element embodiments and with the features of embodiment I30, wherein the control unit is configured to switch an illumination state of the light source at a flashing frequency.

I70. The illumination element according to the preceding embodiment, wherein the flashing frequency is in the range defined by 1 and 200 Hz, preferably by 1 and 150 Hz, further preferably by 1 and 100 Hz.

I71. The illumination element according to any of the preceding illumination element embodiments, wherein the illumination element comprises an identification tag (154).

I72. The illumination element according to the preceding embodiment, wherein the identification tag comprises an active identification tag.

I73. The illumination element according to the penultimate embodiment, wherein the identification tag comprises a passive identification tag.

I74. The illumination element according to any of the preceding illumination element embodiments, wherein the illumination element comprises a heat exchanger (144).

I75. The illumination element according to the preceding embodiment, wherein the illumination element comprises a passive heat exchanger.

I76. The illumination element according to any of the 2 preceding embodiments, wherein the illumination element comprises an active heat exchanger.

I77. The illumination element according to any of the 3 preceding embodiments, wherein the heat exchanger is configured to regulate a temperature of the illumination element.

I78. The illumination element according to the preceding embodiment and with the features of embodiments I30, and I76, wherein the control unit is further configured to control the heat exchanger.

I79. The illumination element according to any of the 2 preceding embodiments and with the features of embodiment I40, wherein the heat exchanger is configured to regulate the temperature based, at least in part, on the temperature sensed by the temperature sensor.

I80. The illumination element according to any of the preceding illumination element embodiments, wherein the illumination element is configured to be connected to a spacer element.

I81. The illumination element according to any of the preceding illumination element embodiments, wherein the illumination element is configured to be used in an assembly according to embodiment A25, wherein the illumination element is configured to be connected to the tube of the transfer assembly.

I82. The illumination element according to any of the preceding illumination element embodiments, wherein the illumination element is configured to be used in an assembly according to embodiment A28, wherein the illumination element is configured to be connected to the secondary channel of the transfer assembly.

A35. The assembly according to any of the preceding assembly embodiments, wherein each of the plurality of illumination elements comprises an illumination element according to any of the preceding illumination element embodiments.

Below illumination apparatus embodiments will be discussed. These are abbreviated by the letter "J" followed by a number. Whenever reference is herein made to the illumination apparatus embodiments, the following embodiments are meant.

J1. An illumination apparatus comprising an illumination element, the illumination element comprising a light source, and at least one connector member connected to the illumination element, wherein the at least one connector member comprises a connector cable and a plug unit, the plug unit configured to be connected to another plug unit.

J2. The illumination apparatus according to the preceding embodiment, wherein the illumination apparatus comprises two connector members connected to the illumination element, wherein each of the two connector members comprises a connector cable and a plug unit, the plug unit configured to be connected to another plug unit.

J3. The illumination apparatus according to any of the preceding illumination apparatus embodiments, wherein the illumination element comprises an illumination element according to any of the preceding illumination element embodiments but without the features of any of embodiments I2 to I8.

J4. The illumination apparatus according to any of the preceding illumination apparatus embodiments, wherein the connector cable comprises any of a hose, a tube, a fabric tube, or a rubber cable.

J5. The illumination apparatus according to any of the preceding illumination apparatus embodiments, wherein the connector cable comprises any of a metal, a polymer, a plastic, an elastomer, a composite material, a coating, a seal, or a gasket.

J6. The illumination apparatus according to any of the preceding illumination apparatus embodiments, wherein the plug unit comprises any of a metal, a polymer, a plastic, an elastomer, a composite material, a coating, a seal, a gasket, a magnetic connection, or a bayonet lock.

Below power supply element embodiments will be discussed. These are abbreviated by the letter "P" followed by a number. Whenever reference is herein made to the power supply element embodiments, the following embodiments are meant.

P1. A power supply element, wherein the power supply element is configured to be used in an assembly according to any of the preceding assembly embodiments.

P2. The power supply element according to the preceding embodiment, wherein the power supply element is configured to be used in an assembly according to embodiment A5, wherein the power supply element comprises an element plug unit, the element plug unit configured to be connected to a connector plug unit of a connector unit of the assembly.

P3. The power supply element according to embodiment P1, wherein the power supply element is configured to be used in an assembly according to embodiment A12, wherein the power supply element is configured to be connected to a connector cable of a connector assembly of the assembly.

P4. The power supply element according to embodiment P1, wherein the power supply element is configured to be used in an assembly according to embodiment A2, wherein the power supply element is configured to be connected to a connector cable of a connector member of the assembly.

P5. The power supply element according to the preceding embodiment, wherein the power supply element is configured to be firmly connected to the connector cable.

P6. The power supply element according to any of the preceding power supply element embodiments, wherein the power supply element is configured to draw energy from a local grid.

P7. The power supply element according to the preceding embodiment, wherein the power supply element is configured to be connected to a connector element (240), wherein the connector element comprises a connector element cable (244) and a connector element plug unit (242).

P8. The power supply element according to the preceding embodiment, wherein the connector element cable is firmly connected to the power supply element.

P9. The power supply element according to any of the 2 preceding embodiments, wherein the connector element plug unit is complementary to a socket unit of the local grid.

P10. The power supply element according to any of the preceding power supply element embodiments, wherein the power supply element comprises a battery.

The battery may generally be understood to correspond to an energy storage component.

P11. The power supply element according to the preceding embodiment, wherein the battery comprises a rechargeable battery.

P12. The power supply element according to any of the 2 preceding embodiments, wherein the battery comprises any of a Li-ion battery, a Li-polymer battery, a solid-state battery, a super capacitor battery, an Ni-Cd battery, an Ni-Metal Hydride battery, a redox flow battery, a sodium ion battery, and a lithium-sulphur solid-state battery.

P13. The power supply element according to any of the 3 preceding embodiments and with the features of embodiment P6, wherein the battery is configured to be charged by the energy drawn from the local grid.

P14. The power supply element according to any of the 4 preceding embodiments, wherein the battery comprises a replaceable battery.

P15. The power supply element according to any of the preceding power supply element embodiments, wherein the power supply element comprises a power supply element data processing unit.

P16. The power supply element according to any of the preceding power supply element embodiments, wherein the power supply element comprises a power supply element memory unit.

P17. The power supply element according to the preceding embodiment and with the features of embodiment P15, wherein the power supply element memory unit is configured to communicate with the power supply element data processing unit.

P18. The power supply element according to any of the preceding power supply element embodiments, wherein the power supply element comprises a power supply element communication unit.

P19. The power supply element according to the preceding embodiment and with the features of embodiment P15, wherein the power supply element communication unit is configured to communicate with the power supply element data processing unit.

P20. The power supply element according to any of the 2 preceding embodiments, wherein the power supply element communication unit is configured to communicate with an external device and/or element.

P21. The power supply element according to any of the 3 preceding embodiments, wherein the power supply element is configured to be used with an assembly according to embodiment A19, wherein at least one of the plurality of illumination elements comprises an illumination element with the features of embodiment I34, wherein the power supply element communication unit is configured to communicate with the at least one illumination element by means of the connector assembly.

P22. The power supply element according to the preceding embodiment, wherein the power supply element, particularly the power supply element communication unit thereof, is configured to receive data from the at least one illumination element.

P23. The power supply element according to the preceding embodiment and with the features of embodiment P20, wherein the power supply element communication unit is configured to send the received data, or at least a part thereof, to the external device and/or element.

P24. The power supply element according to any of the 2 preceding embodiments and with the features of embodiment P19, wherein the power supply element communication unit is configured to send the data to the power supply element data processing unit. P25. The power supply element according to the preceding embodiment, wherein the power supply element data processing unit is configured to perform a defined action based, at least in part, on the data received.

P26. The power supply element according to any of the 2 preceding embodiments and with the features of embodiment P17, wherein the power supply element data processing unit is configured to send the data received to the power supply element memory unit.

P27. The power supply element according to any of the preceding power supply element embodiments and with the features of embodiment P22, wherein the at least one illumination element comprises an illumination element further with the features of embodiment I36, wherein the data received comprises data measured by the sensor, or at least data related thereto.

P28. The power supply element according to any of the preceding power supply element embodiments and with the features of embodiment P21, wherein the power supply element is configured to send data to the at least one illumination element.

P29. The power supply element according to any of the preceding power supply element embodiments, wherein the power supply element is configured to be used in an assembly according to embodiment A14, wherein a density of the power supply element is less than a density of the reaction medium, or at least a part thereof.

P30. The power supply element according to any of the preceding power supply element embodiments, wherein the power supply element comprises a housing.

P31. The power supply element according to the preceding embodiment, wherein the housing comprises any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material.

The material may be rigid or flexible (inflatable/expandable/stretchable).

P32. The power supply element according to any of the 2 preceding embodiments, wherein the housing comprises a plurality of housing sections, such as two housing sections.

P33. The power supply element according to the preceding embodiment, wherein the two housing sections are configured to be releasably attached to each other.

P34. The power supply element according to the preceding embodiment, wherein the two housing sections are configured to be attached to each other by means of a thread connection.

P35. The power supply element according to any of the 2 preceding embodiments, wherein the two housing sections are configured to be attached to each other by means of a bayonet connection.

P36. The power supply element according to any of the preceding power supply element embodiments and with the features of embodiment P30, wherein the housing comprises, at least significantly, any of a spherical, an oval, a cylindrical, an angular, a platonic solid, or a polygonal shape.

P37. The power supply element according to the preceding embodiment and with the features of embodiment P32, wherein the housing comprises, at least significantly, a spherical shape, wherein each of the two housing sections comprises, at least significantly, a hemispherical shape.

P38. The power supply element according to any of the preceding power supply element embodiments and with the features of embodiment P30, wherein the power supply element is configured to be used in an assembly according to embodiment A14, wherein the housing is, at least partially, impermeable and/or inert to the reaction medium.

P39. The power supply element according to any of the preceding power supply element embodiments and with the features of embodiment P10, and wherein a storage capacity of the battery is based, at least in part, on the total illuminated (illuminable) surface area of the plurality of illumination elements in the assembly.

P40. The power supply element according to any of the preceding power supply element embodiments and with the features of embodiment P10, wherein a storage capacity of the battery is in the range defined by 0.005 and 50 kWh per m², preferably by 0.1 and 25 kWh per m², further preferably by 1 and 10 kWh per m² of illuminated surface area of the plurality of illumination elements connected to the power supply element.

P41. The power supply element according to any of the preceding power supply element embodiments, wherein the power supply element is configured to be connected to a spacer element.

P42. The power supply element according to any of the preceding power supply element embodiments, wherein the power supply element is configured to be used in an assembly according to embodiment A25, wherein the power supply element is configured to be connected to the tube of the transfer assembly.

P43. The power supply element according to any of the preceding power supply element embodiments, wherein the power supply element is configured to be used in an assembly according to embodiment A27, wherein the power supply element is configured to be connected to the primary channel of the transfer assembly.

P44. The power supply element according to any of the preceding power supply element embodiments, wherein the power supply element comprises a light source.

P45. The power supply element according to the preceding embodiment, wherein the power supply element comprises a plurality of light sources.

P46. The power supply element according to the preceding embodiment, wherein at least one of the plurality of light sources is configured to indicate a status of the assembly.

P47. The power supply element according to any of the 2 preceding embodiments, wherein the power supply element is configured to be used in an assembly according to embodiment A14, wherein at least one of the plurality of light sources is configured for illumination of the reaction medium, or at least a part thereof.

P48. The power supply element according to any of the preceding power supply element embodiments, wherein the power supply element comprises a heat exchanger.

A36. The assembly according to any of the preceding assembly embodiments and with the features of embodiment A17, wherein the power supply element comprises a power supply element with the features of embodiment P6, wherein the connector assembly is configured to transfer energy drawn from the local grid to any of the plurality of illumination elements.

Below power apparatus embodiments will be discussed. These are abbreviated by the letter "Q" followed by a number. Whenever reference is herein made to the illumination apparatus embodiments, the following embodiments are meant.

Q1. A power apparatus comprising a power supply element and at least one connector member connected to the power supply element, wherein the at least one connector member comprises a connector cable and a plug unit, the plug unit configured to be connected to another plug unit.

Q2. The power apparatus according to the preceding embodiment, wherein the power apparatus further comprises a connector element connected to the power supply element, wherein the connector element comprises a connector element cable and a connector element plug unit, the connector element plug unit configured to be connected to a socket unit of a local grid.

Q3. The power apparatus according to any of the preceding power apparatus embodiments, wherein the power supply element comprises a power supply element according to any of the preceding power supply element embodiments but without the features of any of embodiments P2 to P5.

Q4. The power apparatus according to any of the preceding power apparatus embodiments, wherein the connector cable comprises any of a hose, a tube, a fabric tube, or a rubber cable.

Q5. The power apparatus according to any of the preceding power apparatus embodiments, wherein the connector cable comprises any of a metal, a polymer, a plastic, an elastomer, a composite material, a coating, a seal, or a gasket.

Q6. The power apparatus according to any of the preceding power apparatus embodiments, wherein the plug unit comprises any of a metal, a polymer, a plastic, an elastomer, a composite material, a coating, a seal, a gasket, a magnetic connection, or a bayonet lock.

Q7. The power apparatus according to any of the preceding power apparatus embodiments and with the features of embodiment Q2, wherein the connector element cable comprises any of a hose, a tube, a fabric tube, or a rubber cable.

Q8. The power apparatus according to any of the preceding power apparatus embodiments and with the features of embodiment Q2, wherein the connector element cable comprises any of a metal, a polymer, a plastic, an elastomer, a composite material, a coating, a seal, or a gasket.

Q9. The power apparatus according to any of the preceding power apparatus embodiments and with the features of embodiment Q2, wherein the connector element plug unit comprises any of a metal, a polymer, a plastic, an elastomer, a composite material, a coating, a seal, a gasket, a magnetic connection, or a bayonet lock.

A37. The assembly according to any of the preceding assembly embodiments and with the features of embodiment A17, wherein the power supply element comprises a power supply element with the features of embodiment P10, wherein the connector assembly is configured to transfer energy stored in the battery to any of the plurality of illumination elements.

A38. The assembly according to any of the preceding assembly embodiments, wherein the power supply element comprises the features of embodiment P28, wherein the at least one illumination element comprises an illumination element further with the features of embodiments I33, and I35, wherein the control unit of the at least one illumination element is configured to control the light source based, at least in part, on the data received from the power supply element.

A39. The assembly according to any of the preceding assembly embodiments, wherein the power supply element comprises a power supply element according to any of the preceding power supply element embodiments.

A40. The assembly according to any of the preceding assembly embodiments and with the features of embodiment A14, wherein a density of the assembly is less than a density of the reaction medium.

A41. The assembly according to the preceding embodiment, wherein a ratio of the density of the assembly to a density of the reaction medium is based, at least in part, on a ratio of a volume of the assembly completely immersed in the reaction medium to a total volume of the assembly.

Below system embodiments will be discussed. These are abbreviated by the letter "S" followed by a number. Whenever reference is herein made to the system embodiments, the following embodiments are meant.

S1. A system for illuminating an interior of a container, wherein the system comprises a plurality of assemblies, and wherein each of the plurality of assemblies comprises an assembly according to any of the preceding assembly embodiments.

S2. The system according to the preceding embodiment, wherein the system comprises a spacer assembly configured to allow an arrangement of the plurality of assemblies in a volume of the container.

S3. The system according to the preceding embodiment, wherein the spacer assembly, or at least a part thereof, is configured to maintain a non-zero distance between any two of the plurality of assemblies.

S4. The system according to any of the 2 preceding embodiments, wherein the spacer assembly comprises at least one or a plurality of spacer elements, wherein each spacer element is connected to an illumination element or to a power supply element of an assembly of the plurality of assemblies.

S5. The system according to the preceding embodiment, wherein at least one of the at least one or plurality of spacer elements is connected to two illumination elements, wherein a first of the two illumination elements is comprised in a first of the plurality of assemblies, and a second of the two illumination elements is comprised in a second of the plurality of assemblies.

S6. The system according to any of the 2 preceding embodiments, wherein at least one of the at least one or plurality of spacer elements is connected to two power supply elements, wherein a first of the two power supply elements is comprised in a first of the plurality of assemblies, and a second of the two power supply elements is comprised in a second of the plurality of assemblies.

S7. The system according to any of the 3 preceding embodiments, wherein at least one of the at least one or plurality of spacer elements comprises any of a rope, a line spacer, a chain, a rigid rod, a rigid tube, a metal rod, a plastic rod, a flexible hose, buoyant foam, a ring, or a sphere, such as a permeable sphere.

S8. The system according to any of the preceding system embodiments and with the features of embodiment S4, wherein each spacer element is firmly connected to the respective illumination element or to the power supply element.

S9. The system according to any of the preceding system embodiments and with the features of embodiment S4, but without the features of the preceding embodiment, wherein each spacer element is releasably connected to the respective illumination element or to the power supply element.

S10. The system according to any of the preceding system embodiments, wherein each of the plurality of illumination elements in each of the plurality of assemblies comprises an illumination element according to embodiment I20, wherein the housing comprises, at least significantly, a spherical shape, and wherein a radius of the housing is, at least significantly, identical for each of the plurality of illumination elements.

S11. The system according to any of the preceding system embodiments, wherein the power supply element in each of the plurality of assemblies comprises a power supply element according to embodiment P36, wherein the housing comprises, at least significantly, a spherical shape, and wherein a radius of the housing is, at least significantly, identical for each power supply element.

S12. The system according to any of the preceding system embodiments and with the features of embodiment S4, wherein at least one of the at least one or plurality of spacer elements is configured to be connected to any of a wall and/or a top of the container, an external mounting/fastening, a ceiling, or a crane track.

Below use embodiments will be discussed. These are abbreviated by the letter "U" followed by a number. Whenever reference is herein made to the use embodiments, the following embodiments are meant.

U1. Use of an assembly according to any of the preceding assembly embodiments or a system according to any of the preceding system embodiments in a photobioreactor.

U2. Use according to the preceding embodiment, wherein the photobioreactor comprises the container and the container comprises a reaction medium.

U3. Use according to any of the preceding use embodiments, wherein the container comprises a volume at least as large as 0.1 m³, preferably at least as large as 0.5 m³, further preferably at least as large as 1 m³.

U4. Use according to any of the 3 preceding embodiments, wherein the illumination element comprises a housing made of metal.

U5. Use according to any of the preceding use embodiments, wherein the container is used for algae cultivation, cultivation of phototrophic organisms, cyanobacteria, corals, underwater plants, moss, microalgae, macroalgae, artificial photosynthetic systems, or biomass production.

U6. Use according to the preceding embodiment, wherein the algae comprise any of Chlorella vulgaris, Spirulina platensis, Haematococcus pluvialis, Phaeodactylum tricornutum, Nostoc muscorum, Chlamydomonas reinhardtii, Dunaliella salina, Nannochloropsis, Scenedesmus, Botryococcus braunii, Porphyridium, Synechocystis, Synechococcus, and co-cultures of phototrophic and non-phototrophic organisms.

U7. Use according to any of the preceding use embodiments, wherein the container comprises an agitator, such as a pump, a flow pump, or a gas circulator.

U8. Use according to any of the preceding use embodiments, wherein the container comprises at least one metal appliance.

U9. Use according to any of the preceding use embodiments, wherein the container comprises any of a gas inlet, a gas outlet, a sensor, or harvesting equipment.

U10. Use according to any of the preceding use embodiments, wherein the container is configured for dosing and/or sampling of a reaction medium in the container.

U11. Use according to any of the preceding use embodiments, wherein the assembly comprises an assembly according to embodiment A21, wherein the container is configured to allow the distal illumination element to be connected to a bottom of the container.

Below method embodiments will be discussed. These are abbreviated by the letter "M" followed by a number. Whenever reference is herein made to the method embodiments, the following embodiments are meant.

M1. A method for illuminating an interior of a container, wherein the method comprises providing an assembly according to any of the preceding assembly embodiments or a system according to any of the preceding system embodiments.

M2. The method according to the preceding embodiment, wherein the method comprises arranging the assembly and/or the plurality of assemblies in a volume of the container such that the assembly and/or the plurality of assemblies define a number density of illumination elements in the container.

M3. The method according to the preceding embodiment, wherein the method comprises arranging the assembly and/or the plurality of assemblies such that the number density of illumination elements is, at least significantly, identical to a desired number density of illumination elements in the container.

M4. The method according to the preceding embodiment, wherein the container comprises a reaction medium, and wherein the desired number density is based, at least in part, on a total illuminated surface area per unit volume of the reaction medium.

M5. The method according to the preceding embodiment, wherein the total illuminated surface area is in the range defined by 1 and 30 m², preferably by 1 and 25 m², further preferably by 1 and 20 m², for 1000 L volume of the reaction medium.

M6. The method according to any of the 4 preceding embodiments, wherein the method comprises changing the number density of illumination elements in the container.

M7. The method according to the preceding embodiment, wherein changing the number density of illumination elements comprises changing the number of illumination elements in at least one assembly.

M8. The method according to the preceding embodiment, wherein changing the number density of illumination elements comprises changing the number of illumination elements in a plurality of assemblies.

M9. The method according to any of the 2 preceding embodiments, wherein changing the number of illumination elements in a defined assembly of the at least one assembly or of the plurality of assemblies comprises adding an illumination element to or removing an illumination element from the defined assembly.

M10. The method according to the preceding embodiment, wherein the defined assembly comprises an assembly according to embodiment A2, wherein adding an illumination element to (removing an illumination from) the defined assembly comprises connecting (disconnecting) a plug unit of a connector member, the connector cable of which is connected to the illumination element, to (from) the plug unit of another connector member, the connector cable of which is connected to another illumination element or to the power supply element.

M11. The method according to the penultimate embodiment, wherein the defined assembly comprises an assembly according to embodiment A4, wherein adding an illumination element to (removing an illumination from) the defined assembly comprises connecting (disconnecting) a plug unit of each connector member, the connector cable of which is connected to the illumination element, to (from) the plug unit of another connector member, the connector cable of which is connected to another illumination element or to the power supply element.

M12. The method according to embodiment M9, wherein the defined assembly comprises an assembly according to embodiment A5, wherein adding an illumination element to (removing an illumination from) the defined assembly comprises connecting

(disconnecting) an element plug unit of the illumination element to (from) a connector plug unit of a connector unit.

M13. The method according to embodiment M9, wherein the defined assembly comprises an assembly according to embodiment A6, wherein adding an illumination element to (removing an illumination from) the defined assembly comprises connecting (disconnecting) each of the two element plug units of the illumination element to (from) a connector plug unit of a connector unit.

M14. The method according to any of the 4 preceding embodiments, wherein the method comprises removing an illumination element from the defined assembly, and wherein a result of removing the illumination element is disconnection, from the assembly, of at least one illumination element.

An illumination element may be understood to be disconnected from the assembly if energy cannot be transferred from the power supply element to the illumination element.

M15. The method according to the preceding embodiment, wherein the method comprises connecting the disconnected illumination element to the assembly.

M16. The method according to embodiment M9, wherein the defined assembly comprises an assembly according to embodiment A12, wherein adding an illumination element to (removing an illumination from) the defined assembly comprises connecting (disconnecting) an element plug unit of the illumination element to (from) a connector plug unit.

M17. The method according to any of the preceding method embodiments and with the features of embodiment M6, wherein the method comprises changing the number density based, at least in part, on a total illuminated surface area per unit volume of the reaction medium.

M18. The method according to the preceding embodiment, wherein the method comprises changing the total illuminated surface area per unit volume of the reaction medium by a factor less than 30, preferably less than 25, further preferably less than 20.

M19. The method according to any of the 2 preceding embodiments, wherein the method comprises maintaining the number density constant for at least 10 minutes, preferably at least 30 minutes, further preferably at least 1 hour and then changing the number density.

M20. The method according to the preceding embodiment, wherein the container is used for algae cultivation, wherein the method comprises changing the number density based, at least in part, on a level of growth of the algae or formation of a product in the container.

M21. The method according to the any of the preceding method embodiments and with the features of embodiment M6, wherein the container is used for algae cultivation, wherein the method comprises changing the number density based, at least in part, on a type of the algae.

M22. The method according to any of the preceding method embodiments, wherein the assembly comprises a power supply element according to embodiment P10, wherein the method comprises detecting a low, or no, remaining battery capacity of the battery.

M23. The method according to the preceding embodiment, wherein the method comprises, in response to the detection, replacing the power supply element with another power supply element comprising a power supply element according to any of the preceding power supply element embodiments.

M24. The method according to any of the 2 preceding embodiments, wherein the power supply element with low, or no, remaining battery capacity comprises a power supply element further with the features of embodiment P11, wherein the method comprises charging the battery.

M25. The method according to embodiment M22, wherein the power supply element with low, or no, remaining battery capacity comprises a power supply element further with the features of embodiment P14, wherein the method comprises replacing the battery of the power supply element.

M26. The method according to the preceding embodiment, wherein the power supply element with low, or no, remaining battery capacity comprises a power supply element further with the features of embodiment P11, wherein the method comprises charging the replaced battery.

M27. The method according to any of the preceding method embodiments, wherein the container comprises a reaction medium, and wherein the method comprises immersing, at least partially, the at least one assembly or each of the plurality of assemblies in the reaction medium.

M28. The method according to any of the preceding method embodiments, wherein at least one of the at least one or plurality of assemblies comprises a plurality of illumination elements, and wherein the method comprises cleaning at least one of the plurality of illumination elements.

M29. The method according to the preceding embodiment and with the features of the penultimate embodiment, wherein cleaning the at least one illumination element comprises retrieving, from the reaction medium, the at least one assembly.

M30. The method according to the penultimate embodiment and with the features of embodiment M27, wherein cleaning the at least one of the plurality of illumination elements comprises removing, from the at least one of the at least one or plurality of assemblies, the at least one illumination element, and retrieving the at least one illumination element. M31. The method according to any of the preceding method embodiments and with the features of embodiment M27, wherein each of the at least one or plurality of assemblies comprises a plurality of illumination elements, and wherein the method comprises wrapping any of the plurality of illumination elements in a protective film before immersing in the reaction medium.

M32. The method according to the preceding embodiment and with the features of embodiment M28, wherein cleaning the at least one of the plurality of illumination elements comprises changing the protective film wrapped around the at least one illumination element.

M33. The method according to any of the 2 preceding embodiments, wherein the protective film is, at least partially, inert and/or impermeable to the reaction medium.

M34. The method according to any of the preceding method embodiments, wherein at least one of the at least one or plurality of assemblies comprises an illumination element with the features of embodiment 153, wherein the method comprises determining if the sampling unit comprises a sample.

M35. The method according to the preceding embodiment, wherein the method comprises, in response to the determination that the sampling unit comprises a sample, retrieving the sample from the illumination element.

M36. The method according to any of the preceding method embodiments and with the features of embodiment M9, wherein changing the number of illumination elements comprises adding an illumination element to the defined assembly, wherein the defined assembly comprises a power supply element according to embodiment P10, and wherein the method further comprises determining if a storage capacity of the battery is sufficient.

Sufficiency of the battery capacity may be determined based, at least in part, for example, on the total illuminated surface area after the addition of the illumination element.

M37. The method according to the preceding embodiment, wherein the power supply element comprises a power supply element further with the features of embodiment P14, wherein the method comprises, in response to determining the storage capacity of the battery to be insufficient, replacing the battery with another battery of higher storage capacity.

M38. The method according to any of the preceding method embodiments, wherein the method comprises providing a system according to embodiment S2, and wherein the method further comprises realizing, using the spacer assembly, a defined arrangement of the plurality of assemblies in a volume of the container.

M39. The method according to any of the preceding method embodiments, wherein the method comprises providing a system according to embodiments S10, and S11, wherein the method comprises realizing, at least significantly, a close-packing of the plurality of illumination and power supply elements.

I83. The illumination element according to any of the preceding illumination element embodiments, wherein the illumination element is configured to be used in the use according to any of the preceding use embodiments and/or the method according to any of the preceding method embodiments.

P49. The power supply element according to any of the preceding power supply element embodiments, wherein the power supply element is configured to be used in the use according to any of the preceding use embodiments and/or the method according to any of the preceding method embodiments.

Thus, as described above, a particularly advantageous aspect of the present technology may comprise movable assemblies of illumination elements (e.g., spheres) within an algae suspension to be cultivated, said illumination elements comprising light sources (e.g., LEDs) and other components (e.g., sensors, additive dispensing mechanisms, etc...). The assemblies, or even individual illumination elements thereof, can be removed, added, cleaned, charged, used to add/remove substances/additives, or to measure data (using sensors, as described above) as may be desired.

By employing the assembly of illumination elements, as described above, even large containers and volumes, for example, may be transformed into photobioreactors using internal lighting.

The illumination elements may be relatively small (compared to a volume of the reaction medium/container) and flexible and may not have to be permanently attached to a container. Thus, maintenance and repair of the illumination system as a whole may be simplified. Further, these features of the illumination elements may allow the realization of an internal illumination in a vessel, tank, basin, or reactor for algae cultivation. This may make it possible to convert vessels, tanks, and containers that may have previously been used for other purposes into photobioreactors in a simple manner. This may be of great potential in algae cultivation.

By varying the number of illumination elements in an assembly, or the number of assemblies, in a reaction medium of the container, the amount of light may be varied over the growth cycle of the algae. For example, fewer illumination elements at the beginning when the culture is thin, more illumination elements towards the end when the culture is dense. The illumination elements may be removed from the container as required, making cleaning easy. The illumination elements may be replaced with other illumination elements as required. The new illumination elements may even have different light emissions, depending on the algae's requirements (red, blue light, UV light). Defective illumination elements may be replaced with new illumination elements.

The technical advantages described above may be realized by the illumination elements' design. These illumination elements may be manufactured in large numbers outside of the container/tank, independently of the container/tank, so the advantages and cost savings of series production may be realized. Further technical advantages may be achieved by replacing the illumination elements, which may be small, mobile, and transportable. The technically complex and expensive aspect of overhauling, modernizing, or replacing large containers or equipment may, thus, be reduced.

Advances in LED and battery technology may allow the power and light generation to be packed into the assemblies, in particular into the illumination elements and the power supply elements thereof, without major heat emissions. Highly efficient LEDs such as OLEDs may be particularly suitable.

In other words, embodiments of the present technology may allow the lighting unit to be physically separated from the container, making it technically easier and cheaper to realize scaling. In addition, empty containers may be easily transformed into photobioreactors.

The internal lighting may be generated by relatively small, and flexible illumination elements comprised in movable assemblies that may not need to be permanently attached to the container so that they may later be produced (and further developed) independently (of the container) in large series efficiently and economically.

Scaling-up of cultivation volumes in photobioreactors using embodiments of the present invention may allow a linear relationship of the number (and also costs) of the illumination elements. In other words, for example, to scale the production volume by a factor of two, the number of illumination elements may be a factor of two as well, or at least a constant multiple thereof. The linear relationship may be in contrast to conventional photobioreactors with artificial (internal) lighting, as described above, that may require lighting to be installed for the total cultivation volume of all production units present at a production site for maximum lighting. As a production site may typically comprise several photobioreactor units and each unit may be in a different cultivation state not all units may require maximum lighting at once. Thus, more lighting (and, consequently, possibly greater installation cost) may have to be installed than may be needed. Embodiments of the present technology may allow installation of only as many illumination elements (and, thus, lighting and costs) as may be needed depending, for example, on average production situation requirements. This may provide an advantageous and risk-based approach for building of large-scale industrial mass production units for algae.

An existing container may be optimized for algae (or, generally, biomass) cultivation and/or growth by replacing the illumination elements. This may significantly improve efficiency, as no structural changes to large installations may be necessary.

Embodiments of the present technology may allow any container and/or tank to be used as an algae reactor, regardless of size (for example, from small garden ponds to 100,000-liter systems). The lighting may be regulated by the size of the illumination elements, the LEDs installed, and their color (for example, different for brown algae and green algae). In this way, different types of algae may be cultivated optimally.

Containers that are temporarily used for other things (e.g., in food or wine production) may be converted into photobioreactors simply by adding the assemblies of illumination elements. The assemblies and/or individual illumination elements may be easily removed and cleaning them may be simple. Thus, new systems, volumes, and containers may be used for algae cultivation. The simple scaling of photobioreactors may mean that more valuable materials can be produced economically with the help of algae.

Embodiments of the present technology may be employed in a wide range of possible applications, as previously unused containers, vessels, tanks, and reactors may easily be converted into photobioreactors. The present technology may allow cultivating algae in any container, thus enabling the flexible production of algae on an industrial scale. The present technology may be suitable for closed production facilities or open basins, also outdoors (e.g., pond). Containers that are temporarily not in use may be converted into algae reactors with the help of illumination elements, as described above. It may even be conceivable to cultivate algae in garden ponds or to use them in professional fish farming.

Advantageously, the manufacture and design of the illumination elements and the assemblies may be simple, easy, and efficient. They may be produced in large quantities. The illumination elements may be used in connection with process understanding of the desired algae cultivation. Thus, the present technology may allow the development of precisely fitting illumination elements for the algae, scale, and product desired.

It may also be possible to use the illumination elements as light installations as a design or advertising element in (public) water installations.

Thus, movable assemblies of illumination elements (e.g., spheres) may be used for internal illumination for the cultivation of phototrophic organisms, in particular microalgae in a liquid culture medium. The one or more assemblies of illumination elements may be placed in an existing container, vessel, reactor, or basin, thereby enabling the cultivation of phototrophic organisms without other external illumination. Further, the assembly may contain one or more light sources (e.g., LED) (comprised in the illumination elements as described above) and a power supply element (that may comprise, e.g., a rechargeable battery, a battery, a fuel point, an external power supply via cable, etc.) that may supply the illumination elements with energy.

The assembly of illumination elements may be used for the cultivation of phototrophic organisms, in particular prokaryotic and eukaryotic algae, also in combination with other micro- or macro-organisms in a liquid culture medium (e.g., in an aquarium).

The assembly of illumination elements may be used for the cultivation of corals in aquaria or to promote growth of corals in natural environments where not enough lighting may be present to prevent dying of corals.

The illumination elements may comprise a translucent material and/or may comprise a coating of a transparent material and may be made of a material inert to the cultivation medium. A shape of the illumination elements can be round, oval, angular, a platonic solid, or polygonal. For example, the illumination elements may be spherical in shape and may have a diameter of 40 - 1000 mm (possibly an even larger number).

The light sources may be applied to or incorporated in a transparent or non-transparent material and may be inert with respect to the cultivation medium.

The illumination elements may comprise spheres, and may be screwable and may contain threads that make it possible to connect the halves of the sphere to each other.

A light intensity of, for example, 30-3000 µmol/m²/s may be generated at the surface of the illumination elements in the wavelength range, for example, of 200-1000 nm.

The illumination elements may be modified on the surface in order to achieve better light penetration into the culture medium. The illumination elements may also comprise attachments that may be fitted with a light strip or reflective or fluorescent parts.

Some of the illumination elements may also contain substances (additives) that may be helpful for the growth of microorganisms (e.g., nutrients or COz -providing components). These may, then, comprise special additive illumination elements that may be used for dosing nutrients. The additive illumination elements may also comprise light sources.

Some of the illumination elements may contain sensors and measuring devices that may measure, record, and forward data from the reaction medium or culture, as described above. The use of data may make targeted cultivation possible. Data may be transmitted by a cable of the connector assembly as described above. The illumination elements with sensors may also contain light sources. That is, embodiments of the present invention relate to a photobioreactor with internal lighting, where the internal lighting is provided by assemblies of elements (e.g., "illumination elements", and "power supply elements"). The power supply to the light sources inside the illumination elements may be via a power supply element that may comprise, for example, a rechargeable battery, that may be charged inductively or, preferably, via cable, as described above. The illumination elements may be configured to have adjustable LED lighting such that a wavelength, an intensity, or a flashing frequency of the lighting may be adjusted.

The size and number of illumination elements may be selected according to tank size, algae density, algae type, etc., as described above.

The illumination elements and/or the assemblies may be added and removed. The illumination elements may be removed, for example, via disconnection from an assembly.

Charging of the power supply elements may be achieved outside of the suspension (i.e., reaction) medium either inductively or via cable.

Cleaning of the illumination elements may also be carried out outside of the suspension medium, particularly owing to growth of algae in the suspension medium.

The illumination elements may be configured to allow installation of measuring sensors.

Thus, overall, embodiments of the present technology may make it easy to convert large and "arbitrary" containers into photobioreactors with internal lighting.

An advantageous aspect of the present technology may be that no external electric field or emission element for alternating electromagnetic fields may be needed.

The illumination elements according to the present invention may be of particular advantage for algae cultivation. By changing different features of the illumination elements, growth rates of algae may be improved. Particularly relevant for improved cultivation of algae may be the available surface area on the illumination elements, illuminance, and penetration depth. These aspects may be controlled, for example, by controlling a size of the illumination elements, an intensity of the light source within the illumination element, and a number density of illumination elements deployed within a reactor respectively.

Several advantages may be realized by embodiments of the present technology. Firstly, a simple system comprising a plurality of assemblies, each assembly comprising a plurality of illumination elements, for illuminating the interior of a reactor may be provided. Secondly, a number of illumination elements may be increased depending on growth of the algae. Thirdly, a lot of measurement technology may be realized via the illumination elements (for example, optical density through distance between illumination elements and light sensors installed within the illumination elements). Further exemplarily, temperature sensors, light sensors, etc., may be installed. Fourthly, the assemblies may enable high flexibility to transform any container and/or tank into a photobioreactor.

As described above, the power supply element may comprise a rechargeable battery. Charging may be possible inductively or via cable. Any number of illumination elements may be flexibly connected to the power supply element and separated again. The power supply element may be configured to act as an anchor to the illumination elements as well as for supplying energy to the illumination elements. The power supply to the light source may be via a rechargeable battery of the power supply element that may be charged outside the reactor. The power supply element may be removed from the reaction medium (for cleaning, maintenance, etc.). Energy may also be supplied via the power supply element with external power (i.e., a local grid) via a cable.

The removal of the illumination elements from the reaction medium may also be of advantage in reducing difficulty and complexity of maintenance of the illumination elements. For example, if the sensor, or any other component of the illumination element malfunctions, said sensor or component may be easily replaced. The ability to thus replace the sensor or the component may be of particular advantage, for example, when the number of illumination elements available is low.

The illumination elements may comprise various sensors as described above (together with a light source). Alternatively, or additionally, sensors may also be provided in the power supply element.

Further, the illumination elements may be configured for providing additives to the reaction medium. In other words, the illumination elements may be configured for dosing, e.g., for CO₂ fumigation (such as a CO₂ - stainless-steel pressure illumination element that may comprise a housing made, at least in part, of stainless steel, and that may comprise pressurized CO₂ that may be released in the reaction medium) or for dosing of nutrients, chemicals, or other additives.

Exemplary features of the invention are further detailed in the figures and the description of the figures below.

### Brief Description of Figures

Figure 1 depicts a photobioreactor with internal tube lighting;
Figure 2a depicts an embodiment of an assembly according to the present invention;
Figure 2b depicts another embodiment of an assembly according to the present invention;
Figure 2c depicts yet another embodiment of an assembly according to the present invention;
Figure 3a depicts an embodiment of an assembly according to the present invention in a container;
Figure 3b depicts another embodiment of an assembly according to the present invention in a container;
Figure 4 depicts an embodiment of an illumination element according to the present invention;
Figure 5 depicts an embodiment of an illumination element with a heat exchanger;
Figure 6 depicts an embodiment of an assembly according to the present invention with a matter transfer assembly;
Figure 7 depicts an assembly according to the present invention with a gas outlet;
Figure 8a depicts a side view of an embodiment of a system comprising a plurality of assemblies according to the present invention in a container;
Figure 8b depicts a top view of the embodiment of the system comprising a plurality of assemblies according to the present invention in a container;
Figure 9a depicts a side view of another embodiment of a system comprising a plurality of assemblies according to the present invention in a container;
Figure 9b depicts a top view of the other embodiment of the system comprising a plurality of assemblies according to the present invention in a container;
Figure 10 depicts an embodiment of a system comprising different embodiments of a spacer element according to the present invention;
Figure 11a depicts another embodiment of a system comprising another embodiment of a spacer element according to the present invention;
Figure 11b depicts a top view of an embodiment of a system with a plurality of spacer elements according to the present invention;
Figure 12 depicts an embodiment of a system with a plurality of spacer elements according to the present invention; and
Figure 13 depicts another embodiment of a system with a plurality of spacer elements according to the present invention.

For the sake of clarity, some features may only be shown in some figures, and others may be omitted. However, also the omitted features may be present, and the depicted and discussed features do not need to be present in all embodiments.

### Detailed Description of Figures

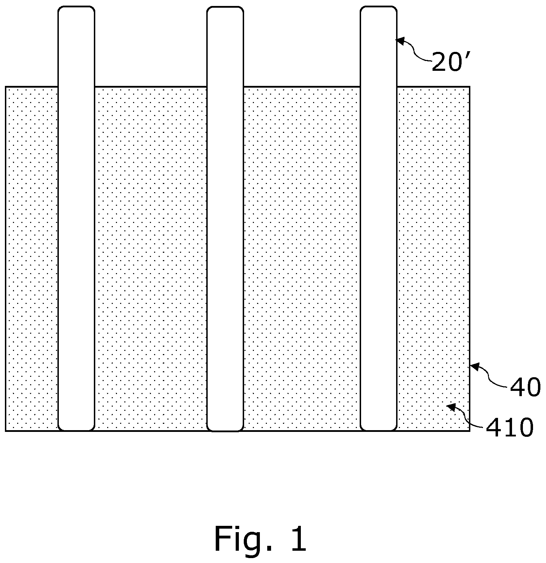

Figure 1 depicts a photobioreactor with internal tube lighting. The photobioreactor may comprise a container 40 comprising a reaction medium 410. One or more lighting tubes 20' may be installed within the container 40 to provide internal lighting. As described above, such a photobioreactor may be subject to certain disadvantages, particularly relating to maintenance and scaling. It is an object of the present invention to provide a photobioreactor that may be easier to maintain and to scale.

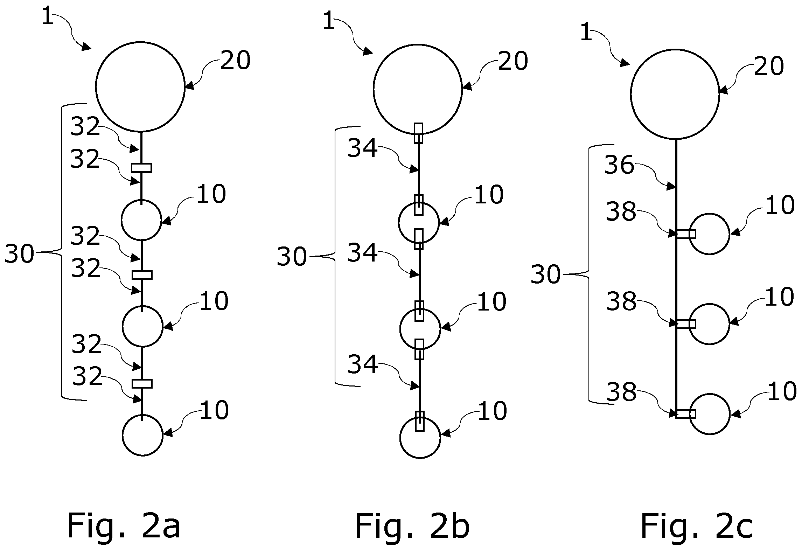

Figure 2 depicts three embodiments of an assembly 1 according to the present invention. Figure 2a depicts a first embodiment of the assembly 1, Figure 2b depicts a second embodiment of the assembly 1, and Figure 2c depicts a third embodiment of the assembly 1 according to the present invention. The assembly 1, according to each of the embodiments depicted in Figure 2, may comprise a plurality of illumination elements 10 and a power supply element 20. Each of the plurality of illumination elements 10 may comprise a light source 110, preferably an LED. The power supply element 20 may be configured to supply energy to each of the plurality of illumination elements 10.

The assembly 1 may further comprise a connector assembly 30 configured to connect the power supply element 20 to each of the plurality of illumination elements 10. The connection may be understood, in particular, to comprise an electrical connection such that electrical energy may be transferred from the power supply element 20 to each of the plurality of illumination elements 10. In other words, at least some, preferably all, of the components of the connector assembly 30 may be configured to conduct electricity.

The connector assembly 30 may be further configured for data transfer. Data transfer may be of advantage in allowing data to be transferred, for example, between any of the plurality of illumination elements 10 and the power supply element 20. Exemplary data that may be transferred may be control data, sensor data, or any other data as described further below.

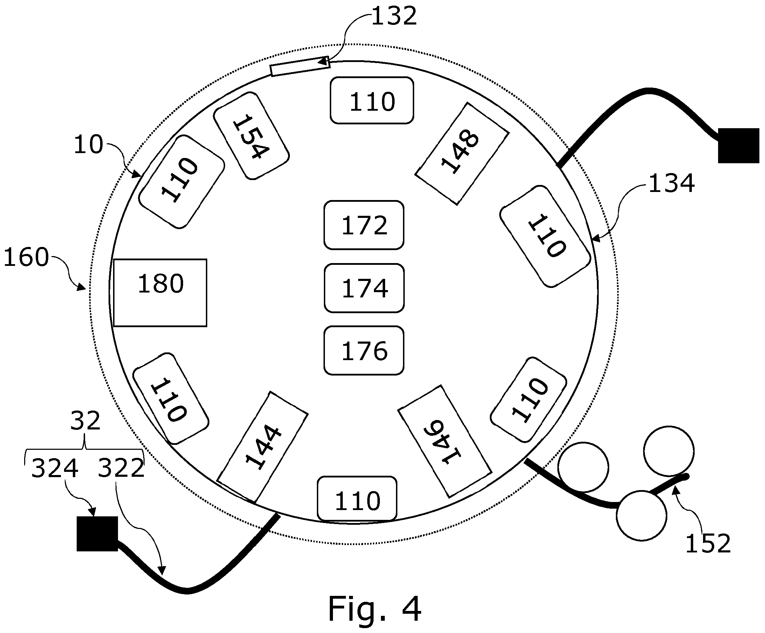

Figure 2a depicts a first embodiment of the assembly 1 comprising a connector assembly 30 with a plurality of connector members 32, each connector member 32 comprising a connector cable 322 and a plug unit 324 (as depicted in Figure 4). The plug unit 324 may be configured to connect to another plug unit 324 of a different connector member 32. Thus, as depicted in Figure 2a, different illumination elements 10 may be connected to and/or disconnected from the assembly 1. The connection between any two of the plug units 324 may be electrically conducting, so as to allow electrical energy to be supplied from the power supply element 10 to each of the plurality of illumination elements 10.

The connector member 32 may be configured to be firmly connected to the respective illumination element 10 or to the power supply element 20.

Figure 2b depicts another embodiment of the assembly 1 comprising a connector assembly 30, wherein the connector assembly 30 comprises a plurality of connector units 34, each connector unit 34 comprising two connector plug units at the ends of the respective connector unit 34. The connector plug units may be configured to connect with complementary element plug units comprised in an illumination element 10 or in a power supply element 20. The illumination element 10 may comprise two element plug units, each of the two element plug units configured to be connected to a connector plug unit of a connector unit 34. In particular, a first of the two element plug unit may be configured to a connector plug unit of a first connector unit 34 and a second of the two element plug units may be configured to be connected to a connector plug unit of a second connector unit 34.

Figure 2c depicts yet another embodiment of the assembly 1 comprising a connector assembly 30, wherein the connector assembly comprises a connector cable 36 to which a plurality of connector plug units 38 may be connected. The connector cable 36 may be connected to the power supply element 20. Each of a plurality of illumination elements 10 may be connected to each of the plurality of connector plug units 38. As may be appreciated each of the plurality of illumination elements 10 may comprise an element plug unit complementary to the connector plug unit 38 to allow the connection between the connector cable and the illumination element 10.

The assembly 1 may comprise a proximal illumination element 10 connected to the power supply element 20 or that may have the power supply element 20 as a direct neighbor (in particular, in case of the embodiment depicted in Figure 2c). The assembly 1 may further comprise a distal illumination element 10 that may correspond to the illumination element 10 farthest (i.e., with the greatest number of illumination elements 10 between the illumination element 10 and the power supply element 20) from the power supply element 20.

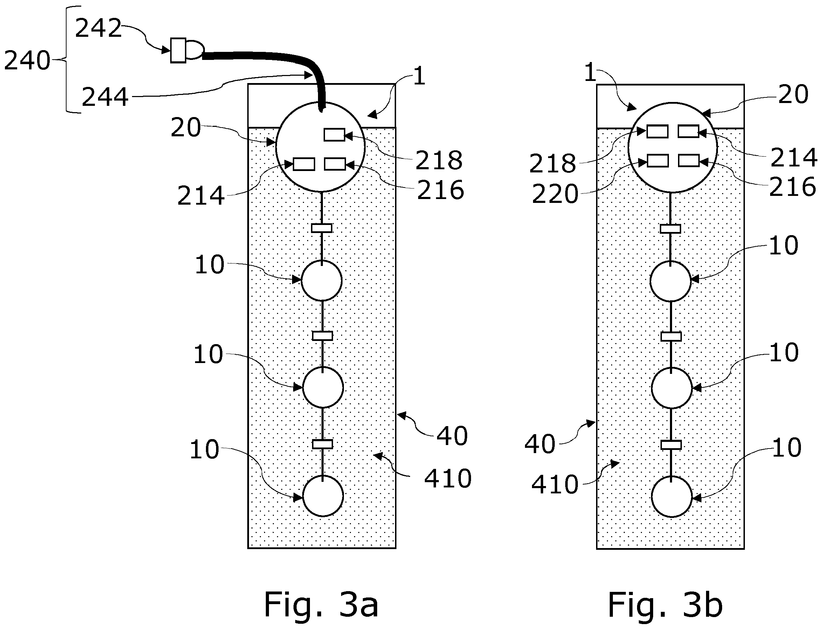

The assembly 1 according to the present invention may be of advantage in illuminating an interior of a container 40, as depicted in Figures 3a and 3b. The container 40 may, in particular, comprise a reaction medium 410, and may be configured for cultivation of biological matter. In particular, the container 40 may be used for cultivation of algae. Introduction of the assembly 1 into the container 40, particularly the reaction medium 410 thereof, may allow conversion of the container 40 to a photobioreactor. Light from the plurality of illumination elements 10 may help promote growth of algae in the container 40.

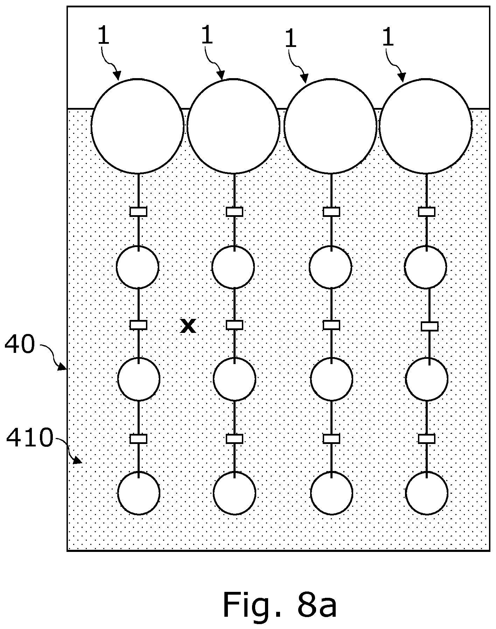

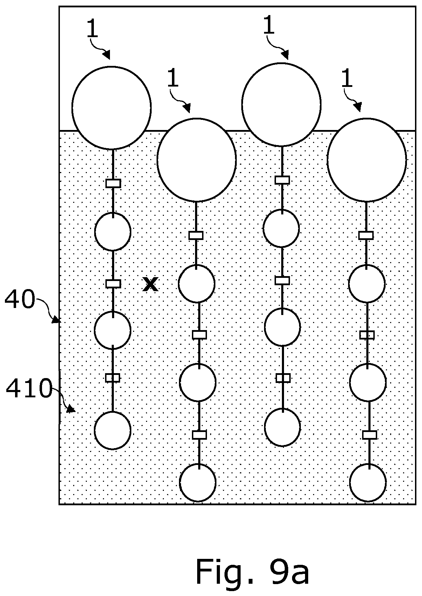

In order to allow the assembly 1 to be arranged within the container 40, a density of the power supply element 20 and each of the plurality of illumination elements 10 may be chosen appropriately. It may be particularly advantageous for the power supply element 20 to additionally function as an anchor and for the plurality of illumination elements 10 to dangle from it into the reaction medium 410, so as to allow a suspension of the illumination elements 10 at different depths in the reaction medium 410 (see Figures 8a and 9a, for example). Thus, a density of the power supply element 20 may be chosen to be, at least substantially, lower than a density of the reaction medium 410. Generally, as algae grows in the reaction medium 410, the density of the reaction medium 410 also grows. Therefore, it may be sufficient to choose a density of the power supply element 20 to be less than a density of fresh water, *ρ* = 1 g/ml (20°C). For example, the density of the power supply element 20 may be chosen to be less than 80%, preferably less than 70%, further preferably less than 50% of the density of fresh water.

The plurality of illumination elements 10 may be desired to be suspended in the reaction medium 410 as depicted in Figure 3a and Figure 3b. A density of the illumination elements 10 may be chosen appropriately to allow such suspension. In particular, a density of at least the distal illumination element 10 may be chosen to be, at least substantially, greater than a density of the reaction medium 410. For example, the density of the distal illumination element 10 may be chosen to be at least 10%, preferably at least 25%, further preferably at least 50% greater than a density of fresh water.

The skilled person understands that the density of at least the distal illumination element 10 and the density of the power supply element 20 may, at least in part, depend on each other. In particular, the density of the power supply element 20 may be chosen such that a fraction of volume of the power supply element 20 floats above a surface of the reaction medium 410 when the plurality of illumination elements 10 and the connector assembly 30 are connected to it. Depending on a value of the fraction desired to float above the surface of the reaction medium 410, the density of the power supply element 20 may be chosen. Generally, a skilled person understands, that the total buoyancy of the assembly 1 may be influenced also by optionally installed spacer elements 60, anchors, and gas injection elements 102, 104, as described further below.

Figures 3a and 3b depict the power supply element 20 and each of the plurality of illumination elements 10 comprising a spherical shape. The spherical shape may be of advantage in allowing a density of the respective element to be "chosen" as a volume of the respective element may be changed by simply changing a diameter of the sphere.

However, any other suitable shape may be used for the illumination element 10 or the power supply element 20. Different shapes that may be used are described further below.

Figure 3a further depicts one embodiment of the power supply element 20 according to the present invention. The power supply element 20 may comprise a connector element 240, the connector element 240 comprising a connector element cable 244 and a connector element plug unit 242. The connector element 240 may allow the power supply element 20 to draw energy from a local grid, for example. The energy drawn from the local grid may then be supplied to each of the plurality of illumination elements 10 by means of the connector assembly 30. The connector element plug unit 242 may, in particular, be complementary to a socket unit of the local grid.

Alternatively, or additionally, the power supply element 20 may comprise a battery 220, as depicted in Figure 3b. The battery 220 may comprise a rechargeable battery. The battery 220 may alternatively, or additionally, be replaceable. A capacity of the battery 220 may be based, at least in part, on a total illuminated (illuminable) surface area of the plurality of illumination elements 10 comprised in the assembly 1. For example, the capacity of the battery 220 may be in the range defined by 0.005 and 50 kWh, preferably by 0.1 and 25 kWh, further preferably by 1 and 10 kWh per m² of illuminated (illuminable) surface area of the plurality of illumination elements 10. The provision of the battery 220 may be of particular advantage if the assembly 1 is deployed in a location where energy supply from the local grid may be intermittent or non-existent.

In some embodiments, the power supply element 20 may comprise both a battery 220 and the connector element 240. This may be of advantage in allowing for continuous supply of energy to the plurality of illumination elements 10 even if the battery 220 runs out. Further, in some embodiments, at least a fraction of the energy drawn from the local grid may be used to charge the battery 220, while the remaining fraction may be used to supply energy to the plurality of illumination elements 10.

Figures 3a and 3b further depict the power supply element 20 comprising a power supply element data processing unit 214. The power supply element data processing unit 214 may be of advantage in performing different data processing functions of the assembly 1. The power supply element may further comprise a power supply element communication unit 216 that may be configured to communicate with an external device such as a remote server and/or a computer. The power supply element 20 may further comprise a power supply element memory unit 218 that may be configured for data storage. The power supply data processing unit 214 may be configured to communicate with the power supply element communication unit 216 and/or the power supply element memory unit 218.

As described above, the connector assembly 30 may be configured for data transfer. In particular, the power supply element communication unit 216 may be configured to communicate with the connector assembly 30. The power supply element communication unit 216 may be configured, for example, to receive data from a sensor 180 of any of the plurality of illumination elements 10, as described further below. The power supply element communication unit 216 may be further configured to send data to any of the plurality of illumination elements 10, wherein a control unit 172 of the receiving illumination element 10 may be configured to control a component of the illumination element 10 based, at least in part, on the received data.

The power supply element memory unit 218 may be configured to store the data received from any of the plurality of illumination elements 10. The power supply element communication unit 216 may be further configured to send the data received from any of the plurality of illumination elements 10 to an external device. For example, the power supply element communication unit 216 may be configured to send data wirelessly or by means of a wired connection to the external device. The connection between the external device and the power supply element 20, and thus the plurality of illumination elements 10, may be of particular advantage in monitoring and/or optimizing the growth of algae in the reaction medium 410. For example, the power supply element 20 may send data relating, at least in part, to an optical density in an environment of a defined illumination element 10. The external device may, in response, send data relating to a wavelength of light to be emitted by the defined illumination element 10 based, at least in part, on a value of the optical density. If, for example, the optical density is higher than a threshold, the wavelength of light may be chosen so as to inhibit further growth of the algae or to boost further growth.

Thus, generally, the power supply element communication unit 216 may be of advantage in allowing communication between the assembly 1, or at least a part thereof, with an external device.

The power supply element data processing unit 214 may additionally, or alternatively, be configured for processing the data received from the sensor 180, and for generating a control signal for the illumination element 10 as described above. The power supply element data processing unit 214 may further be configured for monitoring a remaining capacity of the battery 220 of the power supply element and, for example, if the battery capacity falls below a pre-defined threshold, generate a notification. The generated notification may be sent to the external device by means of the supply element communication unit 216 and may be of advantage in allowing a prompt replacement and/or recharging of the battery 220.

Figure 4 depicts an internal view of an exemplary embodiment of the illumination element 10 according to the present invention. The illumination element 10 may comprise a light source 110. Preferably, the illumination element 10 may comprise a plurality of light sources 110. The light source 110 may comprise, for example, any of a light-emitting diode (LED), a quantum dot, a laser, or any other suitable light source. The light source 110 may be configured to emit, at least significantly, monochromatic radiation. In other words, the light source 110 may be configured to be a narrow-band light source. The monochromaticity of the light source 110 may be of advantage in optimizing the growth of algae as different wavelengths of light may be of benefit at different stages of growth of the algae. For example, light of wavelength in the range 380 - 800 nm may be typical photosynthetically active radiation, whereas light of wavelength in the range 200 - 380 nm may be of advantage in light stress induction or sanitization. Further, light with wavelength in the range 800 - 1000 nm may be of advantage in boosting growth or stress. Thus, generally, it may be of advantage to control a wavelength of light emitted by the light source 110.

The plurality of light sources 110 may each emit light at the same wavelength. This may be of advantage as different illumination elements 10 may be determined to emit light of different wavelengths, each illumination element 10 emitting light, at least significantly, at only one wavelength. Alternatively, the plurality of light sources 110 may emit at different wavelengths. For example, at least some of the plurality of light sources 110 may emit light at a first wavelength (that may be in the range 380 - 800 nm, for example), while at least some other of the plurality of light sources 110 may emit light at a second wavelength (that may be in the range 200 - 380 nm, for example), and yet some other of the plurality of light sources 110 may emit light at a third wavelength (that may be in the range 800 - 1000 nm, for example). However, generally, it may be understood that the plurality of light sources 110 may be appropriately configured (or chosen) to emit light at any number of different wavelengths.

The illumination element 10 may further comprise a control unit 172. The control unit 172 may be configured to control any of the light sources 110. Controlling a light source 110 may be understood to comprise controlling a state (on/off) of the light source 110, a wavelength of light emitted by the light source 110, an intensity of light emitted by the light source 110 (by controlling a current driven through the light source 110, for example), or a flashing frequency of the light source 110. Flashing frequency may be understood to comprise a frequency at which the state of the light source 110 is switched. Flashing light may be of particular advantage for promoting growth of algae. In particular, flashing frequencies in the range defined by 1 and 200 Hz, preferably by 1 and 150 Hz, further preferably by 1 and 100 Hz, may be advantageous.

Any of the light sources 110 may be configured to receive energy from a battery 220 or a connection to the local grid of the power supply element 20 as described above.

An energy consumed by the illumination element 10 may be determined based, at least in part, on an illuminated (or illuminable) surface area of the illumination element 10. For example, if the surface area of illumination is larger, a larger number of light sources 110 may be needed, thus increasing the energy consumption. The illuminated (or illuminable) surface area may be determined, for example, to be equal to the surface area of the illumination element 10. Alternatively, or additionally, the illuminated (or illuminable) surface area may be determined based, at least in part, on a position of the light source 110 (or of each of the plurality of light sources 110) within the illumination element 10 and/or on a level of brightness observed at a defined distance and/or the defined distance from the illumination element 10, or at least a part thereof.

The illumination element 10 may further comprise a sensor 180. The sensor 180 may comprise, for example, a density sensor that may be configured to determine a density of the reaction medium 410 in an immediate vicinity of the illumination element 10. The sensor 180 may be configured to send data to a data processing unit 174 of the illumination element 10. For example, the density sensor may send the data related to the determined density of the reaction medium 410 in an immediate vicinity of the illumination element 10.

The data processing unit 174 may be configured to communicate (i.e., exchange data) with the control unit 172. For example, the data processing unit 174 may send the data related, at least in part, to the density of the reaction medium 410 in the immediate vicinity of the illumination element 10, to the control unit 172.

An advantageous aspect of the illumination element 10 according to the present invention may be the ability to install different sensors 180 within the illumination element 10. These sensors 180 may allow monitoring of an environment around the illumination element 10, thus allowing monitoring the growth, for example, of algae. Further, the sensors 180 may allow monitoring conditions within the illumination element 10 allowing control, for example, of light emitted by the light source 110. For example, the sensor 180 may comprise a temperature sensor, preferably a plurality of temperature sensors, configured to measure a temperature of the reaction medium 410 in the immediate vicinity of the illumination element 10, as well as a temperature inside the illumination element 10. The measured temperatures may be sent to the data processing unit 174.

The data processing unit 174 may be configured to store (for example, in a memory unit also comprised in the illumination element 10), or at least access (for example, from a remote device, via the power supply element 20 as described above), a threshold associated with the sensor 180. The data processing unit 174 may be further configured to carry out a defined action when the data received from the sensor 180 crosses the threshold associated with the sensor 180. For example, the defined action may comprise generating a notification.

The data processing unit 174 may be configured, for example, to send data to the control unit 172 related, at least in part, to switching off of the light source 110 in response to the temperature within the illumination element 10, measured by the temperature sensor 180, exceeding a certain threshold. The certain threshold may be chosen, for example, so as to reduce impact on growth of algae within the container 40. Thus, the data processing unit 174 may allow, at least in part, autonomous control of the illumination element 10.

The illumination element 10 may further comprise a heat exchanger 144. The heat exchanger 144 may be of advantage in regulating a temperature inside the illumination element 10, for example. The heat exchanger 144 may comprise a passive and/or an active heat exchanger. In some embodiments, the illumination element 10 may comprise both a passive and an active heat exchanger. Thus, it may be understood, that the heat exchanger 144 may comprise a plurality of heat exchangers 144. An active heat exchanger 144 may be of particular advantage as a state (on/off) of the heat exchanger may be controlled by the control unit 172. For example, the heat exchanger 144 may be activated when the temperature inside the illumination element 10 exceeds a certain threshold. The control of the active heat exchanger 144 may be similarly mediated by the data processing unit 174, as described above.

The illumination element 10 may comprise a communication unit 176 that may be configured to communicate with the data processing unit 174. The communication unit 176 may receive, for example, data from the data processing unit 174 related, at least in part, to a crossing of the threshold by data received from a sensor 180. Alternatively, or additionally, the communication unit 176 may receive the data received by the data processing unit 174 from the sensor 180 without additional processing and the communication unit 176 may be configured to send the data as is.

The communication unit 176 may further be configured to communicate with an external element. The external element may comprise, for example, the power supply element 20 or another illumination element 10.

Communication between the illumination element 10 and the power supply element 20 or another illumination element 10 may be mediated by the connector assembly 30. In other words, the connector assembly 30 may be configured not only for energy transfer but also for data transfer.

The sensor 180 may further comprise any of a position sensor (for determining a position of the illumination element 10 relative to the container 40 and/or to another illumination element 10), a pH sensor, a pressure sensor, a COz and/or O₂ concentration sensor, a brightness sensor, a viscosity sensor, a turbidity sensor, a density sensor, a conductivity sensor, an impedance sensor, an optical density sensor, a photosynthetically active radiation (PAR) sensor, a camera, a microscope, an IR/Raman-spectroscopy-sensor, a cell potential sensor, a potentiostat sensor, a cell cytometry sensor, a specific ion-sensor (e. g., Na⁺, Cl⁻), a specific nutrient concentrations sensor, a photometer sensor, a proximity sensor (to detect a wall of the container), and a proximity sensor/distance sensor between two illumination elements 10. As may be appreciated, different sensors 180 may allow extracting different information about the illumination element 10 and/or its environment, and the information may be used to optimize conditions for growth of the algae.

Figure 4 further depicts an additive dispensing unit 146. The additive dispensing unit 146 may be configured to dispense an additive into the reaction medium 410. The additive may comprise any of CO₂, H₂O₂, O₃, or any other desired additive. Adding additives may allow further control over growth of algae. For example, ozone (Os) and hydrogen peroxide (H₂O₂) may be used for sanitization or oxidative stress induction purposes.

The illumination element 10 may further comprise a sampling unit 148. The sampling unit 148 may be of advantage in retrieving a sample from the reaction medium 410 for analysis. For example, the sampling unit 148 may comprise a storage tank, that may be coupled to a pump. A sample may be drawn into the storage tank. The sampling unit 148 may further be configured to release a sample stored, for example, in the storage tank. The sample may be analyzed later on to determine, for example, a composition of the sample, that may allow determining the progress of a growth curve of the algae within the container 40.

Figure 4 further depicts the illumination element 10 comprising a fluorescent tag 152. The fluorescent tag 152 may comprise a fluorescent paint, that may allow the illumination element 10 to fluoresce. The fluorescent tag may be of advantage in detecting the illumination element 10 by external stimulation.

The illumination element 10 may further comprise an identification tag 154. The identification tag may comprise any of an RFID tag, an NFC tag, or any other suitable tag. The identification tag 154 may allow for automatic identification of the illumination element 10, for example, via radio-wave transmission. The identification tag 154 may, in particular, allow the power supply element 20 to determine which of the plurality of illumination elements 10 has sent a given data element. Collating the identity of the illumination element 10 with the data received from it, may allow the power supply element 20, or an external device, to receive information about a defined location within the reaction medium 410. This may be of advantage in further controlling, monitoring, or optimizing a spatially-resolved growth of algae in the reaction medium 410.

As may be appreciated by the skilled person, at least some of the different components of the illumination element 10, as described above, may be powered, at least in part, by the power supply element 20.

Figure 4 further depicts a housing 134 of the illumination element 10. The housing 134 may comprise any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material, as described above. Further, the housing 134 may comprise a material that may be rigid or flexible (inflatable/expandable/stretchable). In particular, the housing 134 may comprise a material that is, at least partially, inert and/or impermeable to the reaction medium 410.

The housing 134 may comprise a plurality of sections and the plurality of sections may be releasably attached to each other. This may allow access to the interior of the illumination element 10. The releasable attachment may be provided, for example, by means of a thread connection, or a bayonet connection. The plurality of sections may comprise, for example, 2 sections. If the illumination element 10 comprises a spherical shape, the 2 sections may each comprise a hemispherical shape that may be attached to each other by means of threads provided on an exterior surface of one of the 2 sections and complementary threads provided on an interior surface of the other of the 2 sections.

Alternatively, the plurality of housing sections may comprise 3 housing sections as described above, two of the 3 housing sections comprising substantially hemispherical sections configured to be attached to a third substantially cylindrical section.

At least some of the components of the illumination element 10 described above may be disposed within the housing 134. However, the light source 110 may be disposed within the housing 134 (as depicted in Figure 4), or may be embedded within the housing 134, or may even be arranged on an exterior of the housing 134. For example, the light source 110 may comprise a bead of LEDs that may be wrapped around the housing 134. The light source 110 may, in this case, be understood to be, at least partially, inert and/or impermeable to the reaction medium 410. When the light source 110 is disposed within the housing 134, or is embedded in the housing 134, a material of the housing 134 may be, at least partially, transparent to light emitted by the light source 110. Further, when the sensor 180 comprises a sensor configured to detect a radiative signal (i.e., a signal received via radiation such as electromagnetic radiation), the material of the housing 134 may be, at least partially, transparent to such radiative signals.

The illumination element 10 may further comprise an attachment element 132 that may allow a spacer element 60, as described further below, to be connected to the illumination element 10. For example, the attachment element 132 may allow for magnetic snapping of the spacer element 60 on to the illumination element 10. Or, the attachment element 132 may allow for a thread-based, or a hook-based, attachment to the spacer element 60.

The illumination element 10 may, in some embodiments, be covered with a film 160. The film 160 may comprise, for example, a polyethylene, a polyester, or a plastic film (e.g., PVC, PMMA, Acryl, Nylon, Polycarbonate, Silicone, Latex, transparent polymer). The film 160 may be, at least partially, transparent to light emitted by the light source 110. The film 160 may be replaceable and may allow, for example, to allow cleaning of the illumination element 10 easily. The film 160 may, additionally, serve to protect and/or to water-proof, at least in part, the illumination element 10.

Thus, overall, embodiments of the present technology may provide an illumination element 10 that may allow converting any container 40 into a photobioreactor and that may be simpler, easier to install, and may allow greater monitoring and control over the cultivation process.

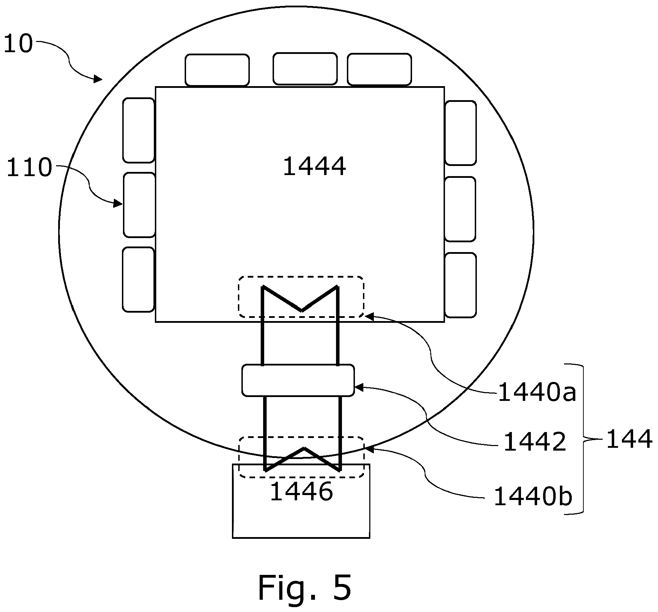

Figure 5 depicts another embodiment of the illumination element 10 comprising an active heat exchanger 144. The depicted embodiment particularly depicts a configuration of a plurality of heat exchanging surfaces 1440a, 1440b in the illumination element 10 (note that the particular embodiment of the illumination element 10 depicted in Figure 5 may also comprise the other components as depicted in the other figures but that are not depicted here). As depicted in Figure 5, the heat exchanging surfaces 1440a, 1440b may be connected to a pump 1442. The pump 1442 may allow pumping, for example, a working fluid through the heat exchanging surfaces 1440a, 1440b. The pumping of the working fluid may thus allow heat flow between a heat source 1444 and a heat sink 1446. The pump 1442 may be controlled by the control unit 172 of the illumination element 10, for example. The heat source 1444 may comprise, for example, a frame and/or a surface on which the light source(s) 110 may be arranged. The frame and/or the surface may comprise a material that has a high thermal conductivity such as aluminum or, generally, any other metal. A further advantage of using aluminum may be a low density allowing for a lower overall weight of the illumination element 10. The thermal conductivity may, for example, be at least 100 W/m/K, preferably at least 150 W/m/K, further preferably at least 200 W/m/K. The heat sink 1446 may, for example, be configured to dissipate heat into the reaction medium 410. Alternatively, or additionally, the heat sink 1446 may comprise a heat storage material that has a high thermal capacity such as lithium nitrate, sodium acetate, sodium acetate trihydrate, or a phase change material (that may, for example, change phase by absorbing heat) that may be configured to store heat received from the heat source 1444. The thermal capacity may, for example, be at least 1 J/g/K, preferably at least 2 J/g/K, further preferably at least 3 J/g/K. The heat storage material may be cooled and/or replaced by retrieving the illumination element 10 from the reaction medium 410, for example.

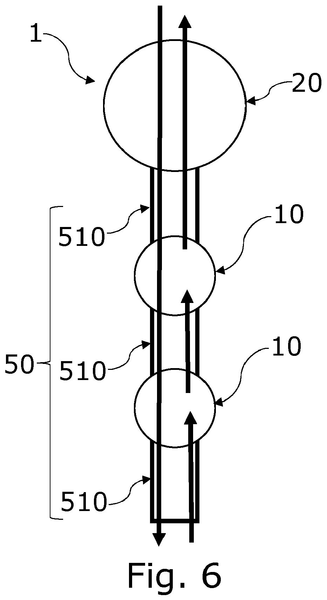

Figure 6 depicts an embodiment of the assembly 1 together with a transfer assembly 50. In particular, the transfer assembly 50 may comprise a plurality of tubes 510 connected between the plurality of illumination elements 10 and/or the power supply element 20. The plurality of tubes 510 may allow a solid, a fluid, a suspension, or an emulsion to be driven through each of the plurality of illumination elements 10 and the power supply element 20. The transfer assembly 50 may further comprise one or more pumps configured to drive any of the solid, the fluid, the suspension, or the emulsion, through the assembly 1.

In particular, the transfer assembly 50 may allow for heat to be transferred. For example, the plurality of tubes 510 may comprise heat exchanging surfaces, similar to the heat exchanging surfaces 1440a, 1440b described above, and a working (or cooling) fluid may be driven through the plurality of tubes 510. Heat from any of the plurality of illumination elements 10 or the power supply element 20 hotter than the working fluid may then be transferred to the working fluid.

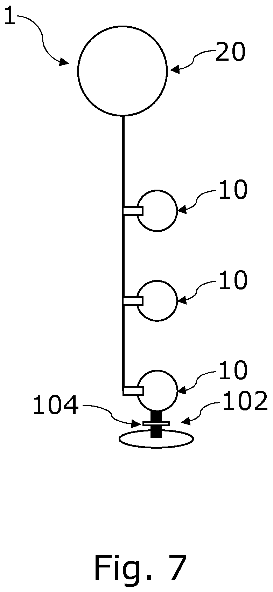

Figure 7 depicts an embodiment of the assembly 1 according to an embodiment of the present invention with the distal illumination element 10 configured to be connected to a bottom of the container. In the depicted embodiment, the distal illumination element 10 comprises a connection element 102 for connection with the bottom of the container. The connection element 102 further comprises a gas inlet 104, although it should be understood that it is not necessary that the gas inlet 104 is provided as a part of the connection element 102, and it could also be provided at another portion of the distal illumination element 10. Generally, it should be understood that the gas inlet 104 and the connection to the bottom of the container are embodiments that are independent from one another, i.e., they can be used in concert (as in Fig. 7), but may also be used independently from one another. By means of the gas inlet, gas can be provided to the container, e.g., air or COz. By the distal illumination element usually being placed at a bottom part or lower part of the container, it will be understood that the gas may thus be introduced into the lower part of the container. By gas usually having a lower density than the medium in the container, the gas may travel upwardly, thus essentially the entire height of the interior of the container will be subjected to the gas.

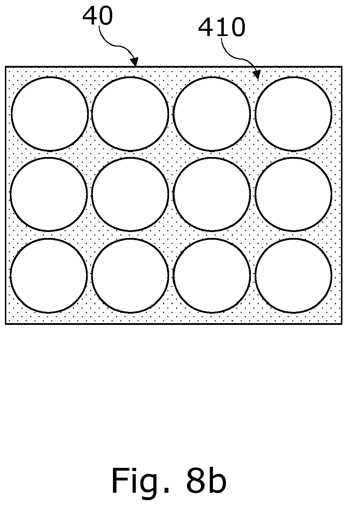

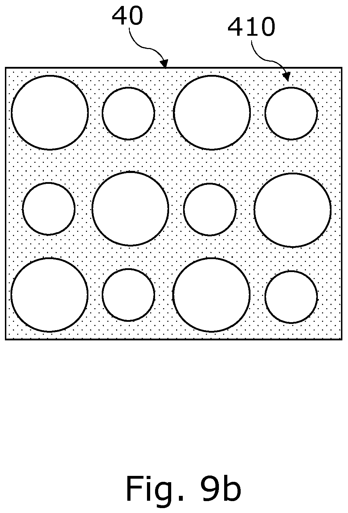

Figures 8 and 9 depict embodiments of a system comprising a plurality of assemblies 1 according to the present invention. In particular, Figure 8 depicts different views of one arrangement of the plurality of assemblies 1 in a container 40, and Figure 9 depicts different views of another arrangement of the plurality of assemblies 1 in a container 40.

Figures 8a and 9a depict side views of two arrangements of the plurality of assemblies in a container 40, whereas Figures 8b and 9b depict the corresponding top views of the arrangements. As depicted in Figure 8a, each of the plurality of assemblies 1 is, at least significantly, identical to any other of the plurality of assemblies 1. Consequently, when suspended in the reaction medium 410 of the container 40, the plurality of assemblies 1 are, at least in the early stages of growth of algae, lined up with each other such that the power supply elements 20 and the illumination elements 10 of the plurality of assemblies 1 are at, at least significantly, identical depths in the reaction medium 410.

Such an arrangement of the plurality of assemblies 1 may, however, be of disadvantage as light emission may vary significantly between the illumination elements 10. For example, consider the location marked 'x' in Figure 8a. At this location, the intensity of light is, at least substantially, lower than an intensity of light in an immediate vicinity of an illumination element 10. As a result, growth of algae in a vicinity of the location marked 'x' may be inhibited compared to growth in the immediate vicinity of the illumination element 10.

An alternative arrangement is depicted in Figure 9a. This arrangement may be considered to correspond, at least significantly, to a close-packing arrangement of the plurality of illumination elements 10. As may be appreciated by the skilled person, such an arrangement may allow a more uniform illumination of the reaction medium 410, and may, thus, promote a more uniform growth of algae in the reaction medium 410.

Further, as the top view in Figure 9b illustrates, individual assemblies 1 may be staggered in two dimensions to, thus, achieve a more uniform illumination of the reaction medium 410.

The staggering of individual assemblies 1, as depicted in Figure 9, may be accomplished, for example, by means of different densities of any of the components of the assembly 1, such as of the power supply element 20 or of any of the plurality of illumination elements 10. It may, however, be of advantage, to vary the density of only one of element, such as the power supply element 20.

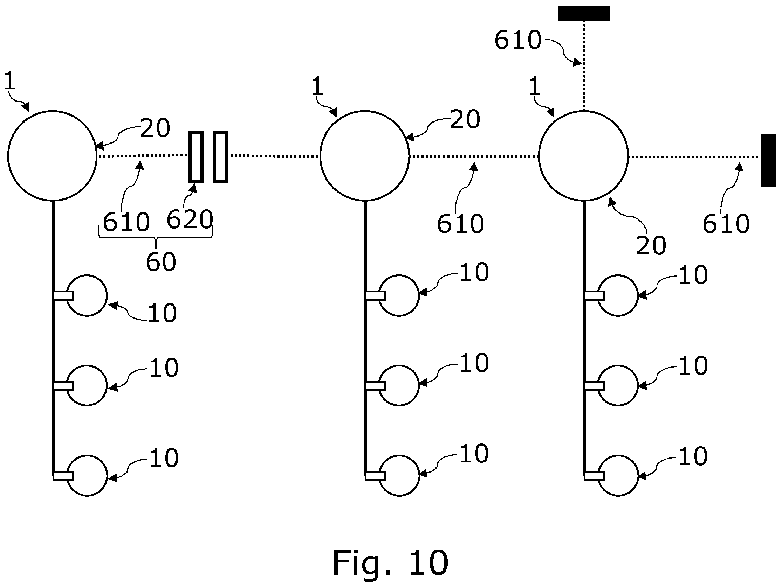

In other embodiments, the system may further comprise a spacer assembly comprising one or more spacer elements 60, indicated as dashed lines in Figures 10 to 13. The spacer elements 60 may be connected between any two illumination elements 10, or between any two power supply elements 20, and may allow a distance between the connected elements to be, at least substantially, different from zero. The spacer elements 60 may be firmly or releasably connected to an illumination element 10 or a power supply element 20.

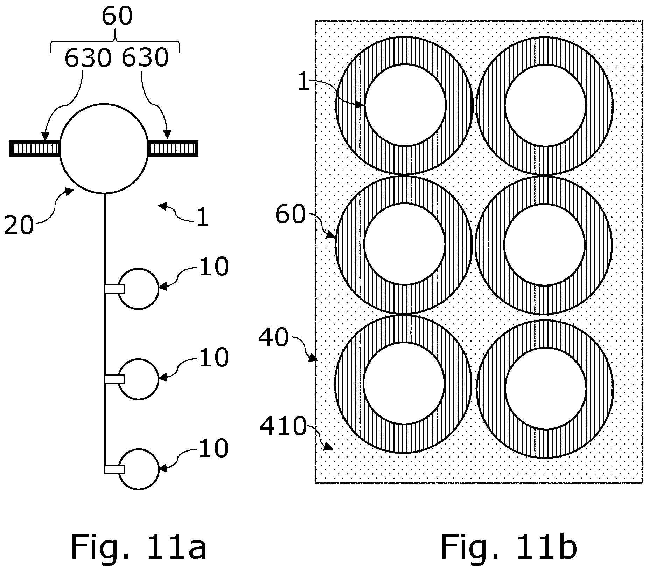

Figure 10 and 11 depict different embodiments of the system according to the present invention with different embodiments of the spacer element 60.

Figure 10 depicts an embodiment comprising a plurality of spacer elements 60, each of the plurality of spacer elements 60 connected to a power supply element 20. The spacer elements 60 may comprise a spacer element connector 610 and a spacer block 620, or in another embodiment may simply comprise a spacer element connector 610. The spacer element 60 may also be configured to allow the assembly 1 to be connected to any of an external mounting/fastening, a wall of the container 40, a ceiling, or to a crane track, as further depicted by connections to the solid blocks in Figure 10.

Alternatively, the spacer element 60 may comprise a plurality of spacer element sections 630, as depicted in Figure 11a, extending on a side of the power supply element 20.

Figure 11b depicts a top view of an embodiment of a system comprising spacer elements 60 according to the embodiment depicted in Figure 11a. As depicted in Figure 11, the spacer elements 60 may allow the plurality of assemblies 1 to stay separated from each other when deployed in a container 40.

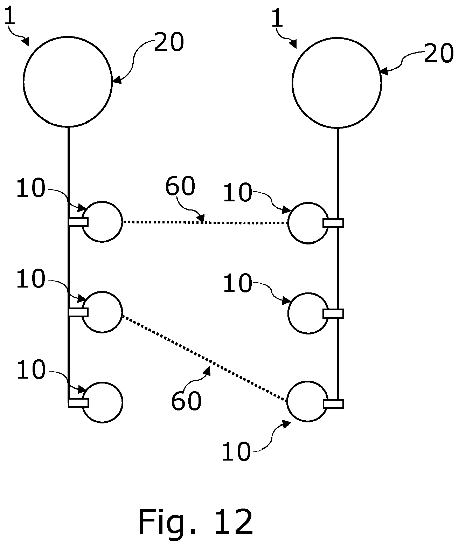

Figure 12 depicts yet another embodiment of the system according to the present invention comprising two assemblies 1 connected by spacer elements 60. As depicted in Figure 12, the spacer elements 60 may be connected between different illumination elements 10, including illumination elements 10 that may be, at least significantly, at the same height, and illumination elements 10 that may be, at least significantly, at different heights.

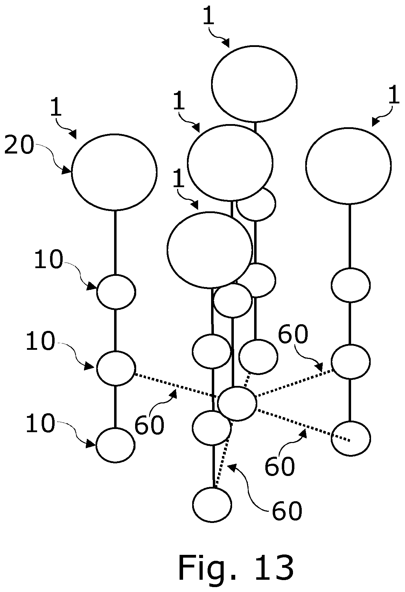

Figure 13 depicts yet another arrangement of a plurality of assemblies 1 in three dimensions, with spacer elements 60 allowing the plurality of assemblies 1 to be arranged such that, at least substantially, a closed-packing configuration of the illumination elements 10 is reached. As described above, such a configuration may be of particular advantage in uniform illumination of the reaction medium 410.

Overall, embodiments of the present technology thus provide elements, systems, and methods, that may allow conversion of any container into a photobioreactor more simply, easily, and efficiently. Further, embodiments of the present technology may allow more robust, simple, and efficient, monitoring and control of the growth process of biological species in the container.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

While in the above, preferred embodiments have been described with reference to the accompanying drawings, the skilled person will understand that these embodiments were provided for illustrative purpose only and should by no means be construed to limit the scope of the present invention, which is defined by the claims.

## Claims

1. Use of an assembly (1) in a photobioreactor, wherein the assembly is adapted for illuminating an interior of a container (40), wherein the assembly comprises
a plurality of illumination elements (10), wherein each illumination element (10) comprises a light source,
a power supply element (20), and
a connector assembly (30) for connecting the power supply element (20) to the plurality of illumination elements (10).

2. Use according to the preceding claim, wherein the connector assembly (30) comprises a plurality of connector members (32), wherein each connector member (32) comprises a connector cable (322) and a plug unit (324), the plug unit (324) configured to be connected to another plug unit, wherein each connector cable (322) is connected to an illumination element (10) or to the power supply element (20).

3. Use according to the preceding claim, wherein each connector cable (322) is firmly connected to the respective illumination element (10) or to the power supply element (20).

4. Use according to any of the preceding claims, wherein the connector assembly is configured, at least in part, to conduct electricity, or wherein the connector assembly is configured, at least in part, for data transfer.

5. Use according to any of the preceding claims, wherein the assembly is configured, at least in part, for matter transfer between any of the plurality of illumination elements and the power supply element.

6. Use according to any of the preceding claims, wherein the container comprises a reaction medium, wherein the assembly is configured to be, at least partially, immersed in the reaction medium, and wherein a density of the power supply element is less than a density of the reaction medium, or at least a part thereof.

7. Use according to any of the preceding claims, wherein the plurality of illumination elements comprises a distal illumination element which is connected only to one other illumination element, wherein the container comprises a reaction medium, wherein the assembly is configured to be, at least partially, immersed in the reaction medium, and wherein a density of the distal illumination element is greater than a density of the reaction medium.

8. Use according to any of the preceding claims, wherein each of the plurality of illumination elements further comprises a sensor (180).

9. Use according to any of the preceding claims, wherein each of the plurality of illumination elements comprises a control unit (172), wherein the control unit is configured to control the light source.

10. Use according to any of the preceding claims, wherein the power supply element is configured to supply energy to the light source(s) of any of the plurality of illumination elements.

11. Use according to any of the preceding claims and with the features of claim 4, wherein the power supply element comprises a power supply element communication unit, wherein at least one of the plurality of illumination elements further comprises a communication unit (176) configured to send and/or receive data by means of the connector assembly of the assembly, wherein the power supply element communication unit is configured to communicate with the at least one illumination element by means of the connector assembly.

12. Use according to any of the preceding claims, wherein any of the plurality of illumination elements and/or the power supply element comprises a heat exchanger.

13. Use of a system in a photobioreactor, wherein the system is adapted for illuminating an interior of a container, wherein the system comprises a plurality of assemblies, and wherein each of the plurality of assemblies comprises
a plurality of illumination elements (10), wherein each illumination element (10) comprises a light source,
a power supply element (20), and
a connector assembly (30) for connecting the power supply element (20) to the plurality of illumination elements (10).

14. Use according to any of the preceding claims, wherein the container is used for algae cultivation, cultivation of phototrophic organisms, cyanobacteria, corals, underwater plants, moss, microalgae, macroalgae, artificial photosynthetic systems, or biomass production.

15. A method for illuminating an interior of a container, wherein the method comprises providing a system adapted for illuminating an interior of a container, wherein the system comprises a plurality of assemblies, wherein each of the plurality of assemblies comprises
a plurality of illumination elements (10), wherein each illumination element (10) comprises a light source,
a power supply element (20), and
a connector assembly (30) for connecting the power supply element (20) to the plurality of illumination elements (10).

16. The method according to the preceding claim, wherein the method comprises arranging the plurality of assemblies in a volume of the container such that the plurality of assemblies define a number density of illumination elements in the container, such that the number density of illumination elements is, at least significantly, identical to a desired number density of illumination elements in the container.

17. The method according to any of the 2 preceding claims, wherein the container comprises a reaction medium, and wherein the method comprises immersing, at least partially, the assembly in the reaction medium, and wherein the method comprises wrapping any of the plurality of illumination elements in a protective film before immersing in the reaction medium.

18. The method according to any of the 3 preceding claims, wherein the system comprises a spacer assembly configured to allow an arrangement of the plurality of assemblies in a volume of the container, and wherein the method further comprises realizing, using the spacer assembly, a defined arrangement of the plurality of assemblies in a volume of the container.
